(19) Europäisches Patentamt
European Patent Office
Office européen des brevets

(11) EP 3 418 298 A1

(12) **EUROPEAN PATENT APPLICATION**

(43) Date of publication:
**26.12.2018 Bulletin 2018/52**

(51) Int Cl.:
*C07K 14/705* (2006.01)

(21) Application number: **17177679.2**

(22) Date of filing: **23.06.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Universität Heidelberg**
**69117 Heidelberg (DE)**

(72) Inventors:
- **Kumar, Varun**
  **69115 Heidelberg (DE)**
- **Nawroth, Peter**
  **69181 Leimen (DE)**
- **Fleming, Thomas**
  **69115 Heidelberg (DE)**
- **Mall, Marcus**
  **69117 Heidelberg (DE)**

(74) Representative: **Patent- und Rechtsanwälte Ullrich & Naumann PartG mbB**
**Schneidmühlstrasse 21**
**69115 Heidelberg (DE)**

# (54) RAGE PROTEINS FOR THE TREATMENT OF FIBROSIS AND DNA DAMAGE MEDIATED DISEASES

(57) The present invention relates to a phosphomimetic RAGE protein comprising an amino acid sequence that is at least 90 % identical to a native mammalian RAGE isoform or fragment thereof wherein at least one serine present in the cytoplasmatic tail of the mammalian RAGE isoform is replaced by a phosphomimetic structure. The invention further relates to a polynucleotide encoding phosphomimetic RAGE protein and a vector comprising the polynucleotide. Finally, the invention relates to a composition comprising carrier and an active pharmaceutical ingredient, selected from the RAGE protein the vector for use in the treatment or prevention of a disease in a patient, wherein the disease is preferably selected from a disease initiated by impaired DNA repair, a disease initiated by increased DNA damage or a disease initiated by increased senescence.

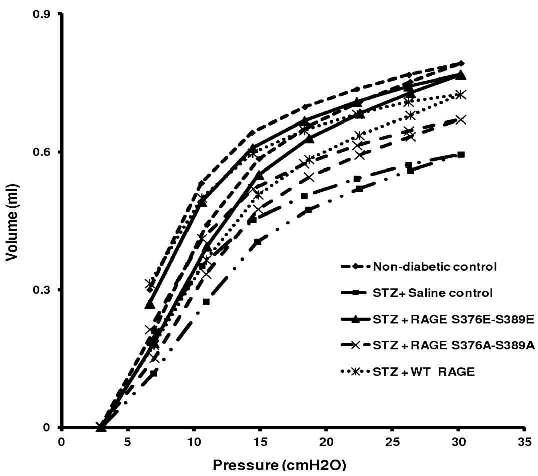


Fig. 1

EP 3 418 298 A1

Printed by Jouve, 75001 PARIS (FR)

SEQUENCE LISTING

<110> Universität Heidelberg

<120> RAGE proteins for the treatment of fibrosis and DNA damage mediated diseases

<130> 6024P1008EP

<160> 22

<170> PatentIn version 3.5

<210> 1
<211> 402
<212> PRT
<213> Mus musculus

<400> 1

```
Met Pro Ala Gly Thr Ala Ala Arg Ala Trp Val Leu Val Leu Ala Leu
1               5                   10                  15

Trp Gly Ala Val Ala Gly Gly Gln Asn Ile Thr Ala Arg Ile Gly Glu
            20                  25                  30

Pro Leu Val Leu Ser Cys Lys Gly Ala Pro Lys Lys Pro Pro Gln Gln
        35                  40                  45

Leu Glu Trp Lys Leu Asn Thr Gly Arg Thr Glu Ala Trp Lys Val Leu
    50                  55                  60

Ser Pro Gln Gly Gly Pro Trp Asp Ser Val Ala Arg Ile Leu Pro Asn
65                  70                  75                  80

Gly Ser Leu Leu Leu Pro Ala Thr Gly Ile Val Asp Glu Gly Thr Phe
                85                  90                  95

Arg Cys Arg Ala Thr Asn Arg Arg Gly Lys Glu Val Lys Ser Asn Tyr
            100                 105                 110

Arg Val Arg Val Tyr Gln Ile Pro Gly Lys Pro Glu Ile Val Asp Pro
        115                 120                 125

Ala Ser Glu Leu Thr Ala Ser Val Pro Asn Lys Val Gly Thr Cys Val
    130                 135                 140

Ser Glu Gly Ser Tyr Pro Ala Gly Thr Leu Ser Trp His Leu Asp Gly
145                 150                 155                 160

Lys Leu Leu Ile Pro Asp Gly Lys Glu Thr Leu Val Lys Glu Glu Thr
                165                 170                 175
```

```
Arg Arg His Pro Glu Thr Gly Leu Phe Thr Leu Arg Ser Glu Leu Thr
            180                 185                 190

Val Ile Pro Thr Gln Gly Gly Thr His Pro Thr Phe Ser Cys Ser Phe
        195                 200                 205

Ser Leu Gly Leu Pro Arg Arg Arg Pro Leu Asn Thr Ala Pro Ile Gln
    210                 215                 220

Leu Arg Val Arg Glu Pro Gly Pro Pro Glu Gly Ile Gln Leu Leu Val
225                 230                 235                 240

Glu Pro Glu Gly Gly Ile Val Ala Pro Gly Gly Thr Val Thr Leu Thr
                245                 250                 255

Cys Ala Ile Ser Ala Gln Pro Pro Pro Gln Val His Trp Ile Lys Asp
            260                 265                 270

Gly Ala Pro Leu Pro Leu Ala Pro Ser Pro Val Leu Leu Leu Pro Glu
        275                 280                 285

Val Gly His Glu Asp Glu Gly Thr Tyr Ser Cys Val Ala Thr His Pro
    290                 295                 300

Ser His Gly Pro Gln Glu Ser Pro Pro Val Ser Ile Arg Val Thr Glu
305                 310                 315                 320

Thr Gly Asp Glu Gly Pro Ala Glu Gly Ser Val Gly Glu Ser Gly Leu
                325                 330                 335

Gly Thr Leu Ala Leu Ala Leu Gly Ile Leu Gly Gly Leu Gly Val Val
            340                 345                 350

Ala Leu Leu Val Gly Ala Ile Leu Trp Arg Lys Arg Gln Pro Arg Arg
        355                 360                 365

Glu Glu Arg Lys Ala Pro Glu Ser Gln Glu Asp Glu Glu Glu Arg Ala
    370                 375                 380

Glu Leu Asn Gln Ser Glu Glu Ala Glu Met Pro Glu Asn Gly Ala Gly
385                 390                 395                 400

Gly Pro


<210>  2
<211>  402
```

<212> PRT
<213> Mus musculus

<400> 2

Met Pro Ala Gly Thr Ala Ala Arg Ala Trp Val Leu Val Leu Ala Leu
1 5 10 15

Trp Gly Ala Val Ala Gly Gly Gln Asn Ile Thr Ala Arg Ile Gly Glu
20 25 30

Pro Leu Val Leu Ser Cys Lys Gly Ala Pro Lys Lys Pro Pro Gln Gln
35 40 45

Leu Glu Trp Lys Leu Asn Thr Gly Arg Thr Glu Ala Trp Lys Val Leu
50 55 60

Ser Pro Gln Gly Gly Pro Trp Asp Ser Val Ala Arg Ile Leu Pro Asn
65 70 75 80

Gly Ser Leu Leu Leu Pro Ala Thr Gly Ile Val Asp Glu Gly Thr Phe
85 90 95

Arg Cys Arg Ala Thr Asn Arg Arg Gly Lys Glu Val Lys Ser Asn Tyr
100 105 110

Arg Val Arg Val Tyr Gln Ile Pro Gly Lys Pro Glu Ile Val Asp Pro
115 120 125

Ala Ser Glu Leu Thr Ala Ser Val Pro Asn Lys Val Gly Thr Cys Val
130 135 140

Ser Glu Gly Ser Tyr Pro Ala Gly Thr Leu Ser Trp His Leu Asp Gly
145 150 155 160

Lys Leu Leu Ile Pro Asp Gly Lys Glu Thr Leu Val Lys Glu Glu Thr
165 170 175

Arg Arg His Pro Glu Thr Gly Leu Phe Thr Leu Arg Ser Glu Leu Thr
180 185 190

Val Ile Pro Thr Gln Gly Gly Thr His Pro Thr Phe Ser Cys Ser Phe
195 200 205

Ser Leu Gly Leu Pro Arg Arg Arg Pro Leu Asn Thr Ala Pro Ile Gln
210 215 220

Leu Arg Val Arg Glu Pro Gly Pro Pro Glu Gly Ile Gln Leu Leu Val
225 230 235 240

Glu Pro Glu Gly Gly Ile Val Ala Pro Gly Gly Thr Val Thr Leu Thr
                245                 250                 255

Cys Ala Ile Ser Ala Gln Pro Pro Pro Gln Val His Trp Ile Lys Asp
            260                 265                 270

Gly Ala Pro Leu Pro Leu Ala Pro Ser Pro Val Leu Leu Leu Pro Glu
        275                 280                 285

Val Gly His Glu Asp Glu Gly Thr Tyr Ser Cys Val Ala Thr His Pro
    290                 295                 300

Ser His Gly Pro Gln Glu Ser Pro Pro Val Ser Ile Arg Val Thr Glu
305                 310                 315                 320

Thr Gly Asp Glu Gly Pro Ala Glu Gly Ser Val Gly Glu Ser Gly Leu
                325                 330                 335

Gly Thr Leu Ala Leu Ala Leu Gly Ile Leu Gly Gly Leu Gly Val Val
            340                 345                 350

Ala Leu Leu Val Gly Ala Ile Leu Trp Arg Lys Arg Gln Pro Arg Arg
        355                 360                 365

Glu Glu Arg Lys Ala Pro Glu Glu Gln Glu Asp Glu Glu Glu Arg Ala
    370                 375                 380

Glu Leu Asn Gln Glu Glu Glu Ala Glu Met Pro Glu Asn Gly Ala Gly
385                 390                 395                 400

Gly Pro

<210> 3
<211> 404
<212> PRT
<213> Homo sapiens

<400> 3

Met Ala Ala Gly Thr Ala Val Gly Ala Trp Val Leu Val Leu Ser Leu
1               5                   10                  15

Trp Gly Ala Val Val Gly Ala Gln Asn Ile Thr Ala Arg Ile Gly Glu
            20                  25                  30

Pro Leu Val Leu Lys Cys Lys Gly Ala Pro Lys Lys Pro Pro Gln Arg
        35                  40                  45

```
Leu Glu Trp Lys Leu Asn Thr Gly Arg Thr Glu Ala Trp Lys Val Leu
    50                  55                  60

Ser Pro Gln Gly Gly Gly Pro Trp Asp Ser Val Ala Arg Val Leu Pro
65                  70                  75                  80

Asn Gly Ser Leu Phe Leu Pro Ala Val Gly Ile Gln Asp Glu Gly Ile
                85                  90                  95

Phe Arg Cys Gln Ala Met Asn Arg Asn Gly Lys Glu Thr Lys Ser Asn
            100                 105                 110

Tyr Arg Val Arg Val Tyr Gln Ile Pro Gly Lys Pro Glu Ile Val Asp
        115                 120                 125

Ser Ala Ser Glu Leu Thr Ala Gly Val Pro Asn Lys Val Gly Thr Cys
    130                 135                 140

Val Ser Glu Gly Ser Tyr Pro Ala Gly Thr Leu Ser Trp His Leu Asp
145                 150                 155                 160

Gly Lys Pro Leu Val Pro Asn Glu Lys Gly Val Ser Val Lys Glu Gln
                165                 170                 175

Thr Arg Arg His Pro Glu Thr Gly Leu Phe Thr Leu Gln Ser Glu Leu
            180                 185                 190

Met Val Thr Pro Ala Arg Gly Gly Asp Pro Arg Pro Thr Phe Ser Cys
        195                 200                 205

Ser Phe Ser Pro Gly Leu Pro Arg His Arg Ala Leu Arg Thr Ala Pro
    210                 215                 220

Ile Gln Pro Arg Val Trp Glu Pro Val Pro Leu Glu Glu Val Gln Leu
225                 230                 235                 240

Val Val Glu Pro Glu Gly Gly Ala Val Ala Pro Gly Gly Thr Val Thr
                245                 250                 255

Leu Thr Cys Glu Val Pro Ala Gln Pro Ser Pro Gln Ile His Trp Met
            260                 265                 270

Lys Asp Gly Val Pro Leu Pro Leu Pro Pro Ser Pro Val Leu Ile Leu
        275                 280                 285

Pro Glu Ile Gly Pro Gln Asp Gln Gly Thr Tyr Ser Cys Val Ala Thr
```

290 295 300

His Ser Ser His Gly Pro Gln Glu Ser Arg Ala Val Ser Ile Ser Ile
305 310 315 320

Ile Glu Pro Gly Glu Glu Gly Pro Thr Ala Gly Ser Val Gly Gly Ser
325 330 335

Gly Leu Gly Thr Leu Ala Leu Ala Leu Gly Ile Leu Gly Gly Leu Gly
340 345 350

Thr Ala Ala Leu Leu Ile Gly Val Ile Leu Trp Gln Arg Arg Gln Arg
355 360 365

Arg Gly Glu Glu Arg Lys Ala Pro Glu Asn Gln Glu Glu Glu Glu Glu
370 375 380

Arg Ala Glu Leu Asn Gln Ser Glu Glu Pro Glu Ala Gly Glu Ser Ser
385 390 395 400

Thr Gly Gly Pro

<210> 4
<211> 404
<212> PRT
<213> Homo sapiens

<400> 4

Met Ala Ala Gly Thr Ala Val Gly Ala Trp Val Leu Val Leu Ser Leu
1 5 10 15

Trp Gly Ala Val Val Gly Ala Gln Asn Ile Thr Ala Arg Ile Gly Glu
20 25 30

Pro Leu Val Leu Lys Cys Lys Gly Ala Pro Lys Lys Pro Pro Gln Arg
35 40 45

Leu Glu Trp Lys Leu Asn Thr Gly Arg Thr Glu Ala Trp Lys Val Leu
50 55 60

Ser Pro Gln Gly Gly Gly Pro Trp Asp Ser Val Ala Arg Val Leu Pro
65 70 75 80

Asn Gly Ser Leu Phe Leu Pro Ala Val Gly Ile Gln Asp Glu Gly Ile
85 90 95

Phe Arg Cys Gln Ala Met Asn Arg Asn Gly Lys Glu Thr Lys Ser Asn

```
            100                 105                 110

Tyr Arg Val Arg Val Tyr Gln Ile Pro Gly Lys Pro Glu Ile Val Asp
        115                 120                 125

Ser Ala Ser Glu Leu Thr Ala Gly Val Pro Asn Lys Val Gly Thr Cys
    130                 135                 140

Val Ser Glu Gly Ser Tyr Pro Ala Gly Thr Leu Ser Trp His Leu Asp
145                 150                 155                 160

Gly Lys Pro Leu Val Pro Asn Glu Lys Gly Val Ser Val Lys Glu Gln
                165                 170                 175

Thr Arg Arg His Pro Glu Thr Gly Leu Phe Thr Leu Gln Ser Glu Leu
            180                 185                 190

Met Val Thr Pro Ala Arg Gly Gly Asp Pro Arg Pro Thr Phe Ser Cys
        195                 200                 205

Ser Phe Ser Pro Gly Leu Pro Arg His Arg Ala Leu Arg Thr Ala Pro
    210                 215                 220

Ile Gln Pro Arg Val Trp Glu Pro Val Pro Leu Glu Glu Val Gln Leu
225                 230                 235                 240

Val Val Glu Pro Glu Gly Gly Ala Val Ala Pro Gly Gly Thr Val Thr
                245                 250                 255

Leu Thr Cys Glu Val Pro Ala Gln Pro Ser Pro Gln Ile His Trp Met
            260                 265                 270

Lys Asp Gly Val Pro Leu Pro Leu Pro Pro Ser Pro Val Leu Ile Leu
        275                 280                 285

Pro Glu Ile Gly Pro Gln Asp Gln Gly Thr Tyr Ser Cys Val Ala Thr
    290                 295                 300

His Ser Ser His Gly Pro Gln Glu Ser Arg Ala Val Ser Ile Ser Ile
305                 310                 315                 320

Ile Glu Pro Gly Glu Glu Gly Pro Thr Ala Gly Ser Val Gly Gly Ser
                325                 330                 335

Gly Leu Gly Thr Leu Ala Leu Ala Leu Gly Ile Leu Gly Gly Leu Gly
            340                 345                 350
```

```
Thr Ala Ala Leu Leu Ile Gly Val Ile Leu Trp Gln Arg Arg Gln Arg
        355                 360                 365

Arg Gly Glu Glu Arg Lys Ala Pro Glu Asn Gln Glu Glu Glu Glu Glu
    370                 375                 380

Arg Ala Glu Leu Asn Gln Glu Glu Glu Pro Glu Ala Gly Glu Ser Ser
385                 390                 395                 400

Thr Gly Gly Pro
```

<210> 5
<211> 1209
<212> DNA
<213> Mus musculus

<400> 5

```
atgccagcgg ggacagcagc tagagcctgg gtgctggttc ttgctctatg gggagctgta      60

gctggtggtc agaacatcac agcccggatt ggagagccac ttgtgctaag ctgtaagggg     120

gcccctaaga agccgcccca gcagctagaa tggaaactga acacaggaag aactgaagct     180

tggaaggtcc tctctcccca gggaggcccc tgggacagcg tggctcgaat cctccccaat     240

ggttccctcc tccttccagc cactggaatt gtcgatgagg ggactttccg gtgtcgggca     300

actaacaggc gagggaagga ggtcaagtcc aactaccgag tccgagtcta ccagattcct     360

gggaagccag aaattgtgga tcctgcctct gaactcacag ccagtgtccc taataaggtg     420

gggacatgtg tgtctgaggg aagctaccct gcagggaccc ttagctggca cttagatggg     480

aaacttctga ttcccgatgg caaagaaaca ctcgtgaagg aagagaccag gagacaccct     540

gagacgggac tctttacact gcggtcagag ctgacagtga tccccaccca aggaggaacc     600

catcctacct tctcctgcag tttcagcctg ggccttcccc ggcgcagacc cctgaacaca     660

gcccccatcc aactccgagt cagggagcct gggcctccag agggcattca gctgttggtt     720

gagcctgaag gtggaatagt cgctcctggt gggactgtga ccttgacctg tgccatctct     780

gcccagcccc ctcctcaggt ccactggata aaggatggtg caccccttgcc cctggctccc    840

agccctgtgc tgctcctccc tgaggtgggg cacgaggatg agggcaccta tagctgcgtg     900

gccacccacc ctagccacgg acctcaggaa agccctcctg tcagcatcag ggtcacagaa     960

accggcgatg aggggccagc tgaaggctct gtgggtgagt ctgggctggg tacgctagcc    1020

ctggccttgg ggatcctggg aggcctggga gtagtagccc tgctcgtcgg ggctatcctg    1080

tggcgaaaac gacaacccag gcgtgaggag aggaaggccc cggaagaaca ggaggatgag    1140

gaggaacgtg cagagctgaa tcaggaagag gaagcggaga tgccagagaa tggtgccggg    1200

ggaccgtaa                                                            1209
```

<210> 6
<211> 1221
<212> DNA
<213> Homo sapiens

<400> 6
atggcagccg gaacagcagt tggagcctgg gtgctggtcc tcagtctgtg gggggcagta 60

gtaggtgctc aaaacatcac agcccggatt ggcgagccac tggtgctgaa gtgtaagggg 120

gcccccaaga aaccacccca gcggctggaa tggaaactga acacaggccg gacagaagct 180

tggaaggtcc tgtctcccca gggaggaggc ccctgggaca gtgtggctcg tgtccttccc 240

aacggctccc tcttccttcc ggctgtcggg atccaggatg aggggatttt ccggtgccag 300

gcaatgaaca ggaatggaaa ggagaccaag tccaactacc gagtccgtgt ctaccagatt 360

cctgggaagc cagaaattgt agattctgcc tctgaactca cggctggtgt tcccaataag 420

gtggggacat gtgtgtcaga gggaagctac cctgcaggga ctcttagctg gcacttggat 480

gggaagcccc tggtgcctaa tgagaaggga gtatctgtga aggaacagac caggagacac 540

cctgagacag ggctcttcac actgcagtcg gagctaatgg tgaccccagc ccggggagga 600

gatccccgtc ccaccttctc ctgtagcttc agcccaggcc ttccccgaca ccgggccttg 660

cgcacagccc ccatccagcc ccgtgtctgg gagcctgtgc ctctggagga ggtccaattg 720

gtggtggagc cagaaggtgg agcagtagct cctggtggaa ccgtaaccct gacctgtgaa 780

gtccctgccc agccctctcc tcaaatccac tggatgaagg atggtgtgcc cttgcccctt 840

ccccccagcc ctgtgctgat cctccctgag atagggcctc aggaccaggg aacctacagc 900

tgtgtggcca cccattccag ccacgggccc caggaaagcc gtgctgtcag catcagcatc 960

atcgaaccag gcgaggaggg gccaactgca ggctctgtgg gaggatcagg gctgggaact 1020

ctagccctgg ccctggggat cctgggaggc ctggggacag ccgccctgct cattggggtc 1080

atcttgtggc aaaggcggca acgccgagga gaggagagga aggccccaga aaaccaggag 1140

gaagaggagg agcgtgcaga actgaatcag gaggaggaac ctgaggcagg cgagagtagt 1200

actggagggc ctccgcggat a 1221

<210> 7
<211> 7
<212> PRT
<213> Simian virus 40

<400> 7

Pro Lys Lys Lys Arg Lys Val
1 5

<210> 8

<211> 16
<212> PRT
<213> Homo sapiens

<400> 8

Lys Arg Pro Ala Ala Thr Lys Lys Ala Gly Gln Ala Lys Lys Lys Lys
1 5 10 15

<210> 9
<211> 9
<212> PRT
<213> Homo sapiens

<400> 9

Pro Ala Ala Lys Arg Val Lys Leu Asp
1 5

<210> 10
<211> 11
<212> PRT
<213> Homo sapiens

<400> 10

Arg Gln Arg Arg Asn Glu Leu Lys Arg Ser Pro
1 5 10

<210> 11
<211> 38
<212> PRT
<213> Homo sapiens

<400> 11

Asn Gln Ser Ser Asn Phe Gly Pro Met Lys Gly Gly Asn Phe Gly Gly
1 5 10 15

Arg Ser Ser Gly Pro Tyr Gly Gly Gly Gly Gln Tyr Phe Ala Lys Pro
20 25 30

Arg Asn Gln Gly Gly Tyr
35

<210> 12
<211> 42
<212> PRT
<213> Homo sapiens

<400> 12

Arg Met Arg Ile Glx Phe Lys Asn Lys Gly Lys Asp Thr Ala Glu Leu
1 5 10 15

Arg Arg Arg Arg Val Glu Val Ser Val Glu Leu Arg Lys Ala Lys Lys

20 25 30

Asp Glu Gln Ile Leu Lys Arg Arg Asn Val
35 40

<210> 13
<211> 8
<212> PRT
<213> Homo sapiens

<400> 13

Val Ser Arg Lys Arg Pro Arg Pro
1 5

<210> 14
<211> 8
<212> PRT
<213> Homo sapiens

<400> 14

Pro Pro Lys Lys Ala Arg Glu Asp
1 5

<210> 15
<211> 8
<212> PRT
<213> Homo sapiens

<400> 15

Pro Gln Pro Lys Lys Lys Pro Leu
1 5

<210> 16
<211> 12
<212> PRT
<213> Mus musculus

<400> 16

Ser Ala Leu Ile Lys Lys Lys Lys Lys Met Ala Pro
1 5 10

<210> 17
<211> 5
<212> PRT
<213> Influenza virus

<400> 17

Asp Arg Leu Arg Arg
1 5

<210> 18

<211> 7
<212> PRT
<213> Influenza virus

<400> 18

Pro Lys Gln Lys Lys Arg Lys
1 5

<210> 19
<211> 10
<212> PRT
<213> Hepatitis D virus

<400> 19

Arg Lys Leu Lys Lys Lys Ile Lys Lys Leu
1 5 10

<210> 20
<211> 10
<212> PRT
<213> Mus musculus

<400> 20

Arg Glu Lys Lys Lys Phe Leu Lys Arg Arg
1 5 10

<210> 21
<211> 20
<212> PRT
<213> Homo sapiens

<400> 21

Lys Arg Lys Gly Asp Glu Val Asp Gly Val Asp Glu Val Ala Lys Lys
1 5 10 15

Lys Ser Lys Lys
20

<210> 22
<211> 17
<212> PRT
<213> Homo sapiens

<400> 22

Arg Lys Cys Leu Gln Ala Gly Met Asn Leu Glu Ala Arg Lys Thr Lys
1 5 10 15

Lys

## Description

### FIELD OF THE INVENTION

The present invention relates to RAGE proteins such as phosphomimetic RAGE proteins and their use in the treatment of DNA damage mediated diseases in particular fibrosis.

### BACKGROUND OF THE INVENTION

Any discussion of the background art throughout the specification should in no way be considered as an admission that such art is widely known or forms part of common general knowledge in the field.

Every cell of the human body encounters tens to thousands of DNA damages every day (Jackson and Bartek, 2009; Ciccia and Elledge, 2010), of which DNA double strand breaks (DSBs) are the most cytotoxic form of DNA lesions. These lesions, if left unrepaired, lead to various disorders such as cancer, neurodegeneration, fibrosis and immunodeficiency (Jackson and Bartek, 2009; Aparicio et al., 2014; O'Driscoll, 2012).

Normally, cells respond to these breaks by a complex series of events commonly known as the DNA damage response (CDDR) which detect, signal and repair DSB by either an error-free homologous recombination repair (HRR) pathway or error-prone non-homologous end joining repair pathway (NHEJ) (Branzei and Foiani, 2008) DSBs lead to the activation of the DSB-sensing ATM kinase, which then phosphorylates several downstream targets including the histone variant H2AX and the effector kinase CHK2 (Matsuoka et al., 2007).

Thus, on-demand orchestration of temporal and spatial repair signaling plays an important role in preserving the genetic integrity. Impairment of DNA repair, leading to an accumulation of DNA DSBs can result in some of the most devastating diseases of the western society, including neurodegeneration, pulmonary fibrosis and cancer.

RAGE is known as the receptor for advanced glycation end products (AGEs) (Schmidt et al., 1992). AGEs are the product of non-enzymatic addition of sugar moieties to proteins. In healthy tissue, RAGE is expressed at a low basal level. However, the up-regulation of RAGE has been associated with a diverse range of pathological events from atherosclerosis to Alzheimer's disease (Bierhaus et al., 2005; Bierhaus and Nawroth, 2009), in which the detrimental effect of RAGE has been viewed as ligand-dependent. Pulmonary tissue expresses the highest basal levels of RAGE, specifically within type 1 alveolar epithelial cells, suggesting that RAGE may play a critical role in lung which is distinct from other tissues expressing RAGE (Demling et al., 2006; Fehrenbach et al., 1998). However, the exact physiological function of RAGE in the lung has yet to be fully understood.

Consistent with the traditionally held view of RAGE as a ligand-dependent, pro-inflammatory mediator of fibrosis, He et al. reported that homozygous RAGE deficient mice (RAGE-/-) were resistant to bleomycin induced lung injury, with enhanced survival and lower fibrotic scores. It was shown that the protein levels of the pro-fibrotic cytokines, TGFß1 and PDGF, in bronochoalveolar lavage fluid were not increased in RAGE-/- following treatment with bleomycin (He et al., 2007).

Different therapeutic approaches addressing the pro-inflammatory function of RAGE have been proposed with RAGE. In these approaches, an isoform of RAGE, termed soluble RAGE is used. This isoform contains the only the extracellular region of RAGE. In this form, soluble RAGE is capable of binding RAGE ligands, prior to their interaction with full-length RAGE. Due to this capacity, soluble RAGE has been proposed and marketed to act as a decoy receptor which can prevent the deleterious effects which arise as a consequence of full-length RAGE activation (Bierhaus et al., 2005; Bierhaus and Nawroth, 2009).

In a recently published document (Kumar *et al,* 2015) the inventors could show that RAGE is either constitutively or inducibly localized to the nucleus. Nuclear-RAGE is recruited to the site of DNA damage via an ATM signaling cascade. Furthermore, RAGE interacts with MRN complex to participate in the nucleolytic processing of DNA ends and simultaneously it protects ssDNA by facilitating the loading of single stranded DNA binding proteins on it. Genetic ablation *in vivo* in mice or knockdown of RAGE *in vitro* leads to failure of homology directed repair, resulting in accumulation of DNA double stranded breaks (DSBs) and enhanced pro inflammatory cytokine milieu. Unrepaired DNA coupled with inflammation contributes to tissue fibrosis, oncogenesis and thereby perturbs organ physiology.

In the Kumar et al. 2015 it is shown that RAGE can translocate to the nucleus and specifically within the lung, where it accumulates in areas of DSB, and becomes phosphorylated at the serines 376 and 389 by the DNA-DSB sensor kinase, 'ATM'.

### SUMMARY OF THE INVENTION

The present invention is, inter alia, based on the surprising finding that a replacement of at least one serine residue in the cytoplasmatic tail (CT) of murine RAGE and human RAGE with a phosphomimetic structure, in particular a phosphomimetic amino acid leads to a significant increase in efficiency of RAGE mediated DNA repair. Moreover, such phosphomimetic RAGE administered to diabetic mice with lung fibrosis does not only promote a stop of the process leading to fibrosis but also induces remission of fibrosis and almost normalization of the lung function. More importantly, this remission of fibrosis was also confirmed for other disease-affected tissues such as e.g. nerve and kidney cells.

Thus, according to the first aspect the invention provides a phosphomimetic RAGE protein comprising an amino acid sequence that is at least 90 % identical to a native mammalian RAGE isoform or fragment thereof wherein at least one serine present in the cytoplasmatic tail (CT) of the mammalian RAGE isoform is replaced by a phosphomimetic structure.

The relevant serine residues replaced by phosphomimetic structures are in particular the targets of RAGE phosphorylation by the ATM signaling cascade. The improved effect in DNA repair of the phosphomimetic RAGE is at least partially caused by an increased nuclear import of the phosphomimetic RAGE relative to wild type RAGE. Therefore, according to a second aspect, the invention provides a fusion protein of a RAGE protein comprising an amino acid sequence that is at least 90 % identical to a native mammalian RAGE isoform or fragment thereof fused to at least one nuclear targeting structure.

According to a third aspect, the invention provides an isolated polynucleotide with a nucleic acid sequence encoding the phosphomimetic RAGE according to the first aspect or the fusion protein according to the second aspect. This isolated polynucleotide is in particularly useful for *in vitro* expression of the RAGE protein according to the first aspect or the RAGE fusion protein according to the second aspect. Alternatively, the isolated polynucleotide may be used for in vivo expression of the RAGE protein, i.e. gene therapy.

For gene therapy, the isolated polynucleotide is in particular introduced into a vector, either for transient transfection and expression of the protein in the patient's cells or for stable integration into the genome of the patient's cells. Thus, according to a fourth aspect, invention provides a vector comprising a polynucleotide according to the third aspect and a promoter.

For treating patients with diseases such as diseases initiated by impaired DNA repair, a disease initiated by increased DNA damage or a disease initiated by increased senescence, either the phosphomimetic RAGE protein itself or a vector comprising a polynucleotide encoding the phosphomimetic RAGE protein maybe used as an active pharmaceutical ingredient (API). In both cases, the API has to be transported into the cells to be treated. For transport, generally, a carrier is required.

Thus, according to a fifth aspect the invention provides a composition comprising carrier and an active pharmaceutical ingredient, selected from a phosphomimetic RAGE protein according to the first aspect, and a vector according to the fourth aspect, for use in the treatment or prevention of a disease in a patient. The disease is preferably selected from a disease initiated by impaired DNA repair, a disease initiated by increased DNA damage or a disease initiated by increased senescence.

According to a sixth aspect the invention provides a method of purification of a RAGE protein with a polyhistidine-tag, comprising the steps of:

- providing a first solution comprising the RAGE protein with a polyhistidine-tag and contaminant proteins;
- subjecting the first solution to a Ni-NTA chromatography and eluting a second solution containing the RAGE protein; and
- subjecting the second solution to a heparin chromatography and eluting a third solution containing the RAGE protein.

## FIGURES

Embodiments of the invention will now be described, by way of example only, with reference to the accompanying drawings in which:

Fig. 1 shows that reconstitution of full-length mRAGE normalizes lung function in diabetic mice. WT non-diabetic and diabetic mice were transduced either with saline control, AAV2/8-RAGE$^{WT}$, AAV2/8-RAGE$^{S376ES389E}$, or AAV2/8-RAGE$^{S376A-S389A}$ virions. Four weeks after transduction, the pulmonary function of the mice was assessed by determination of the pressure-volume curves using the FlexiVent system. Data represent group averages (8-10 mice per group) of at least three independent measurements.

Fig. 2 shows that the phosphomimetic mutant of mRAGE improves the survival ratio in reperfusion injury of the kidney. Diabetic mice pretreated for four weeks with AAV2/8-RAGE$^{S376E-S389E}$ virions showed an increased survival ratio compared to diabetic mice pretreated with saline or empty virion controls over 5-7 days after injury. Data represent relative lethality after reperfusion injury in control vs diabetic mice. STZ: Streptozotocin; N=8

Fig. 3 shows that ATM-mediated phosphorylation of RAGE plays an important role for nuclear accumulation of RAGE. (A) Human RAGE serves as downstream target of activated ATM signaling cascade after induction of DNA DBS by Bleomycin and CPT. The treatment with λ-phosphatase reverses the RAGE phosphorylation. Specific inhibition of ATM prevents phosphorylation of RAGE whereas the general kinase inhibitor Nu7026 could not inhibit RAGE phosphorylation. Moreover, the S391 of human RAGE serves as phosphoacceptor in DNB DSB repair signaling as indicated by the loss of phosphorylation in the hRAGE$^{S389A}$ mutant. Phosphorylated hRAGE is detected via ATM-phosphospecific antibody α-S/TQ. α-FLAG serves as loading control for hRAGE-FLAG. CPT: Camptothecin, N=3.(B) The pre-sensitized lung adenocarcinoma cells were treated with DMSO alone or camptothecin (1 μM for 60 min) induced phosphorylation of hRAGE as well as its co-localization with DNA DSB repair foci marked by γH2AX. The nucleus was stained using DAPIAn antibodies directed to the respective targets as indicated in the figure.

Fig. 4 shows that ATM-mediated phosphorylation of RAGE is decreased in diabetes. RAGE serves as downstream target of the activated ATM signaling cascade. Diabetic mice exhibit a reduced RAGE phosphorylation compared to the irradiated mice. DNA DBS repair mechanisms are induced as indicated by the increased signal obtained in diabetic and irradiated samples for λH2AX. In contrast to the irradiated samples, RAGE is less phosphorylated in diabetic samples, although RAGE protein levels are equal.

Fig. 5 shows that the phosphomimetic mutant of RAGE improves the nerve regeneration. The neuropathy analysis of diabetic mice transduced with AAV2/8-RAGE$^{S376E-S389E}$ virions dramatically improves nerve function. Error bars: ± SEM, N=6-8; p-value was calculcated in an unpaired, two-tailed Student's t-test.

Fig. 6 shows that the phosphomimetic mutant of RAGE reduces renal fibrosis and DNA damage on STZ induced diabetes. (A) Mice were transduced with AAV2/8-RAGE$^{S376E-S389E}$ virions. Kidney tissue stained with Masson's trichrome staining revealed that treatment with the phosphomimitic RAGE resolves the accumulated ECM and hence improved the overall kidney function. (B) Immunofluorescence microscopy of STZ induced diabetic tissue showed a nuclear localization of the DNA DSB repair marker γH2AX. Nuclear localization of γH2AX was absent in tissue transduced with the phosphomimetic RAGE mutant. Scale bar, 50 μm (top panel) and 10 μm (bottom panel), Negative control: secondary antibody only.

Fig. 7 shows a comparative expression and purification of murine RAGE. Left: Top: Schematic overview of the expression and purification of the recombinant RAGE protein. The respective vector construct is transformed into *E.coli* cells. Transformed cells are cultured in TB. Protein purification is performed on Ni-NTA. The obtained purified fractions are dialysed and stored. Bottom Analysis of the purified protein fractions on an SDS-PAGE gel stained with CBB. The purity of WT RAGE (lane 1), RAGE$^{S376A}$ (lane 2), RAGE$^{S389A}$ (lane 3) and RAGE$^{S376A-S389A}$ (lane 4) is shown. A prominent band at 55 kDa is visible in all tested fractions corresponding to recombinant RAGE. Additional bands with higher or lower molecular mass can be seen throughout all analyzed fractions representing contaminations purified together with the recombinant protein.

Fig. 8 shows the expression and purification of ultrapure RAGE. Left: Schematic overview of the expression and purification of the recombinant RAGE protein. The respective vector construct is transformed into *E.coli* cells. Transformed cells are cultured in TB. Protein purification is performed on Ni-NTA. The obtained purified fractions are applied on a Heparin Agarose column for further purification. The resulting ultrapure fractions are applied to a further purification step using a Sephacryl S200 column. The obtained ultrapure protein fractions are dialyzed and stored. Right: Analysis of the purified protein fractions on an SDS-PAGE gel stained with CBB. The purity of WT RAGE (lane 1), RAGE$^{S376A}$ (lane 2), RAGE$^{S389A}$ (lane 3) and RAGE$^{S376A-S389A}$ (lane 4) and $^{RAGES376E-S389}$E (lane 5) is shown. A prominent band at 55 kDa is visible in all tested fractions corresponding to recombinant RAGE. No additional bands with higher or lower molecular mass can be seen in all tested samples, suggesting a high purity of the protein fractions.

## DETAILED DESCRIPTION OF THE INVENTION

In order to provide a clear and consistent understanding of the specification and claims, and of the scope to be given such terms, the following definitions are provided.

### Definitions

A **"peptide"** as used herein relates to a chain of amino acids connected by peptide bonds. A peptide may be composed of any number of amino acids of any type, preferably naturally occurring amino acids, which, preferably, are linked by peptide bonds. In particular, a peptide comprises at least 3 amino acids, preferably at least 5, at least 7, at least 9, at least 12, or at least 15 amino acids. Furthermore, there is no upper limit for the length of a peptide. However, preferably, a peptide according to the invention does not exceed a length of 500 amino acids, more preferably it does not exceed a length of 300 amino acids; even more preferably it is not longer than 250 amino acids. Thus, the term "peptide" includes "oligopeptides", which usually refer to peptides with a length of 2 to 20 amino acids and "polypeptides" with at least 60, at least 80, preferably at least 100 amino acids.

The term **"polypeptide"** as used herein refers to a peptide. The terms "polypeptide" and "protein" are used interchangeably. The polypeptides and proteins as used herein include chemically synthesized proteins as well as naturally synthesized proteins which are encoded by genes. The polypeptides or proteins may be obtained from a natural source, such as human blood or produced in cell culture as recombinant proteins.

The term **"protein"** as used herein relates to may contain one or more polypeptide chains. Proteins with one and one polypeptide chain are often expressed on one polypeptide chain from one gene and cleaved post translation only. Thus, the terms "protein" and "polypeptide" are often used interchangeably.

The term **"protein domain"** or **"domain"** as used herein refers to a region of a protein that can fold into a stable three-dimensional structure independently of the rest of the protein. This structure may maintain a specific function associated with the domain's function within the intact protein, including enzymatic activity, creation of a recognition motif for another molecule, or provision of the necessary structural components for a protein to exist in a particular environment. Protein domains are usually evolutionarily conserved regions of proteins, both within a protein family and within other protein superfamilies that require similar functions.

The term **"fusion protein"** according to the invention relates to proteins created through the joining of two or more genes, cDNAs or sequences that originally coded for separate proteins/peptides. The genes may be naturally occurring in the same organism or different organisms or may synthetic polynucleotides.

The term **"patient"** as used herein denotes a mammal, in particular a human with brain cancer, who is to be treated.

As used herein, the term **"mammal"** refers to any mammal, including, but not limited to, mammals of the order Rodentia, such as mice and hamsters, and mammals of the order Logomorpha, such as rabbits, Carnivora, including Felines (cats) and Canines (dogs), Artiodactyla, including Bovines (cows) and Swines (pigs) or of the order Perssodactyla, including Equines (horses), Primates, Ceboids, or Simoids (monkeys) or of the order Anthropoids (humans and apes).

The relatedness between two amino acid sequences or between two nucleotide sequences is described by the parameter "identity". For purposes of the present invention, the degree of sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et a/., 2000, Trends Genet. 16: 276-277), preferably version 3.0.0 or later. The optional parameters used are gap open penalty of 10, gap extension penalty of 0.5, and the EBLOSUM62 (EMBOSS version of BLOSUM62) substitution matrix. The output of Needle labeled "longest identity" (obtained using the no brief option) is used as the percent identity and is calculated as follows:

$$(\text{Identical Residues x 100})/(\text{Length of Alignment - Total Number of Gaps in Alignment}).$$

For purposes of the present invention, the degree of sequence identity between two nucleotide sequences is determined using the Needleman-Wunsch algorithm (Needleman and Wunsch, 1970, supra) as implemented in the Needle program of the EMBOSS package (EMBOSS: The European Molecular Biology Open Software Suite, Rice et a/., 2000, supra), preferably version 3.0.0 or later. The optional parameters used are gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

$$(\text{Identical Desoxyribonucleotides x 100})/(\text{Length of Alignment - Total Number of Gaps in Alignment})$$

For example an identity of at least 90 % includes 90 %, 91 %, 92 %, 93 %, 94 %, 95 %, 96 %, 97 %, 98 % or even 99% identity to the subject sequence. Typically, similar sequences will include the same residues in positions that are relevant for the function of the peptide or polynucleotide, such as active site residues or glycosylated amino acids, however though may include any number of conservative amino acid substitutions.

The term **"carrier"** according to the invention relates to molecules useful for delivery of a cargo into cells of a patient, in particular an active pharmaceutical ingredient, e.g. a polynucleotide or protein. Carriers may covalently or non-covalently bind to the cargo or may encapsulate the cargo.

The term **"recombinant"** when used in reference to a cell, nucleic acid, protein or vector, indicates that the cell, nucleic acid, protein or vector has been modified by the introduction of a heterologous nucleic acid or protein or the alteration of a native nucleic acid or protein, or that the cell is derived from a cell so modified. Thus, for example, recombinant cells express genes that are not found within the native (non-recombinant) form of the cell, or express native genes at different levels or under different conditions than found in nature.

An **"isolated"** polynucleotide (e.g., an RNA, DNA or a mixed polymer) or peptide according to the invention is one which is substantially separated from other cellular components which naturally accompany a native human sequence or protein, e.g., ribosomes, polymerases, the chromosome, other RNA molecules and proteins. The term embraces a nucleic acid sequence or protein which has been removed from its naturally occurring environment, and includes recombinant or cloned DNA isolates and chemically synthesized analogs or analogs biologically synthesized by heterologous systems.

The transitional term **"comprising",** which is synonymous with "including," "containing," or "characterized by," is inclusive or open-ended and does not exclude additional, unrecited elements or method steps. The transitional phrase "consisting of" excludes any element, step, or ingredient not specified in the claim, except for impurities ordinarily associated therewith. When the phrase **"consists of"** appears in a clause of the body of a claim, rather than immediately following the preamble, it limits only the element set forth in that clause; other elements are not excluded from the claim as a whole. The transitional phrase "consisting essentially of" limits the scope of a claim to the specified materials or steps "and those that do not materially affect the basic and novel characteristic(s)" of the claimed invention. "A 'consisting essentially of claim occupies a middle ground between closed claims that are written in a 'consisting of' format and fully open claims that are drafted in a 'comprising' format."

## Phosphomimetic RAGE

According to the first aspect the invention provides a phosphomimetic RAGE protein comprising an amino acid sequence that is at least 90 % identical to a native mammalian RAGE isoform or fragment thereof wherein at least one serine present in the CD of the mammalian RAGE isoform is replaced by a phosphomimetic structure.

RAGE is a highly conserved protein among the higher multicellular organisms from mice to humans. The gene that encodes RAGE consists of eleven different exons and is located on chromosome 6 in humans and on chromosome 17 in mice, and is located close to the major histocompatibility complex (MHC) III in both species (Sugaya et al., 1994).

Structurally, RAGE belongs to the immunoglobulin superfamily, and the protein consists of an N-terminal signal peptide (amino acids 1-22 in human RAGE), a V-type immunoglobulin-like domain (amino acids 23-116 in human RAGE), two tandem C-type immunoglobulin-like domains (amino acids 124-221 and 227-317, respectively in human RAGE), a single transmembrane domain (amino acids 343-363 in human RAGE), and a short C-terminal intracellular cytoplasmic tail (amino acids 364-404 in human RAGE).

The combined variable and C1 (VC1) domain is mainly involved in ligand binding while the C2 domain is mainly involved in fine tuning of ligand binding and specificity of ligand recognition. The variable domain recognizes ligands due to its inherent three-dimensional structures such as β-sheets and fibrils (Bierhaus, Humpert, Morcos, et al., 2005; Moser, Herold, & Schmidt, 2006; Schmidt, Yan, Yan, & Stern, 2000; S. F. Yan et al., 2007).

The transmembrane domain allows anchoring and spanning of the receptor in the cell membrane. The small cytoplasmic tail is mainly involved in linking the receptor to intracellular signaling pathways (Kislinger et al., 1999; Neeper et al., 1992; Schmidt et al., 1992). RAGE undergoes various post-translational modifications such as glycosylation, ubiquititnation, and phosphorylation (Bannister & Kouzarides, 2011) (177,). Depending upon the degree of these modifications, its molecular weight can range from 48 to 64 KDa or greater.

The ligand binding V, C1 and C2 domains are also involved in DNA-binding. Therefore, without wanting to be bound to theory, it is suggested that these domains are essential for RAGE's function in DNA repair. Accordingly, the phosphomimetic RAGE protein according to the invention preferably comprises at least the VC1 domain. In this regard, it is noted that the most highly conserved fragment of mammalian RAGE is formed by amino acids 40 to 241 of SEQ ID NO: 1, which includes a part of the V domain and the complete C1 domain. According to one embodiment of the first aspect, the native mammalian RAGE isoform or fragment thereof contains the amino acid sequence of amino acids 40 to 241 of SEQ ID NO: 1.

According to a further embodiment the phosphomimetic RAGE protein comprises the VC1 and C2 domain. Moreover, the phosphomimetic replacement of at least one serine to a phosphomimetic structure occurs in the cytoplasmic tail (CT). Accordingly, the phosphomimetic RAGE protein comprises at least a part of the CT. According to a preferred embodiment, the phosphomimetic RAGE protein comprises the complete CT.

According to one embodiment, the phosphomimetic RAGE contains the V domain, the C1 domain, the C2 domain, transmembrane domain and the CT.

According to one embodiment the phosphomimetic RAGE protein comprises an amino acid sequence that is at least 95 % identical to a native mammalian RAGE isoform or fragment thereof. Preferably, the amino acid sequence is at least 98 % identical to a native mammalian RAGE isoform or fragment thereof.

A phosphomimetic structure according to the invention relates to any moiety that appears chemically similar to a phosphorylated amino acid. Commonly, phosphate groups are added to serines, tyrosines and threonines. Thus, a phosphomimetic structure due to similarities in structural and chemical properties can or may be recognized as a constantly phosphorylated serine, a tyrosine or a threonine and can or may have the same effect as a constantly phosphorylated serine, tyrosine or threonine.

The phosphomimetic structures for replacing the one or more serines in the cytoplasmatic domain of RAGE may be the same or different moieties. The phosphomimetic structures may be natural or synthetic amino acids or any other molecule resembling a phosphorylated serine. In phosphomimetic RAGE the at least one serine is preferably replaced by a phosphomimetic amino acid. The phosphomimetic amino acid may be glutamic acid or aspartic acid. According to one embodiment, the phosphomimetic structure is glutamic acid.

The phosphomimetic RAGE contains phosphomimetic structures, in particular in the positions of serines phosphorylated by the ATM signaling cascade. As shown in Kumar et al. 2015, induction of DNA double strand breaks (DSB) by bleomycin or camptothecin, activating the ATM signaling cascade leads to a phosphorylation of murine RAGE at serines 376 and 389 (with SEQ ID NO: 1 as reference) and to a nuclear accumulation of murine RAGE in the nucleus. Moreover, a phosphomimetic RAGE with a replacement of these serines by glutamic acids has an increased effect in the reduction of pulmonary fibrosis as shown in Example 1. In human RAGE an equivalent to murine Ser389 is found in position 391 (with SEQ ID NO: 3 as reference). Ser376 is not conserved in humans. The inventors could now show that induction of DNA DSBs by bleomycin or camptothecin leads to a phosphorylation of human RAGE at serine Ser391 and also to a nuclear accumulation of human RAGE in the nucleus (see Example 4 and in particular Fig. 3). Accordingly, this provides evidence that the effect of nuclear localization and phosphorylation of Ser389 and optionally Ser376 by the ATM signaling cascade is conserved for all mammalian RAGE. Accordingly, the increased pharmacological effect on remission of fibrosis can be reasonably be generalized to phosphomimetic human RAGE and other mammalian RAGE proteins.

With reference to the examples, the phosphomimetic RAGE may for example be derived from murine RAGE. In this case the amino acid sequence is at least 95 % identical to SEQ ID NO: 1 or a fragment thereof. Preferably, the phosphomimetic RAGE is at least 98 % identical, more preferably at least 99 % identical to murine RAGE identified by SEQ ID NO: 1 or a fragment thereof. The fragment of murine RAGE may for example contain amino acids 40 to 214 and 364 to 404 of SEQ ID NO: According to one embodiment the fragment of murine RAGE contains amino acids 23 to 221 and 364 to 404 of SEQ ID NO: 1. According to one embodiment the fragment of murine RAGE contains amino acids 23 to 317 and 364 to 404 of SEQ ID NO: 1. According to one embodiment the fragment of murine RAGE contains amino acids 23 to 404 of SEQ ID NO: 1.

The phosphomimetic RAGE protein derived from murine RAGE may contain a phosphomimentic structure that replaces Ser376 or Ser389. Without wanting to be bound to theory it is assumed that already a replacement of one of the two serines leads to an increase in activity and/or an increase in nuclear localization. However, as shown in the examples, preferably both Ser376 and Ser389 are replaced by phosphomimetic structures. Ser376 and Ser389 are preferably independently replaced by phosphomimetic amino acid(s), such as aspartic acid and glutamic acids. More preferably, Ser376 and Ser389 are both replaced by glutamic acids. Thus, according to one embodiment the phosphomimetic RAGE comprises an amino acid sequence that is at least 95 % identical, preferably at least 98 % identical, more preferably at least 99 % identical to SEQ ID NO: 2.

The phosphomimetic RAGE may alternatively be derived from human RAGE. In this case, the amino acid sequence is at least 95 % identical to SEQ ID NO: 3 or a fragment thereof. Preferably, the phosphomimetic RAGE is at least 98 % identical, more preferably at least 99 % identical to human RAGE identified by SEQ ID NO: 3 or a fragment thereof. The fragment of human RAGE may for example contain amino acids 40 to 214 and 364 to 404 of SEQ ID NO: According to one embodiment the fragment of murine RAGE contains amino acids 23 to 221 and 364 to 404 of SEQ ID NO: 3. According to one embodiment the fragment of human RAGE contains amino acids 23 to 317 and 364 to 404 of SEQ ID NO: 3. According to one embodiment the fragment of human RAGE contains amino acids 23 to 404 of SEQ ID NO: 3. Thus, according to one embodiment the phosphomimetic RAGE comprises an amino acid sequence that is at least 95 % identical, preferably at least 98 % identical, more preferably at least 99 % identical to SEQ ID NO: 4.

The phosphomimetic RAGE protein may be also be fused to other peptides, such as affinity tags, half-life prolonging peptides or targeting peptides.

## Fusion protein

The relevant serine residues replaced by phosphomimetic structures are in particular the targets of RAGE phosphorylation by the ATM signaling cascade. The improved effect in DNA repair of the phosphomimetic RAGE and, consequently, in the reduction of e.g. pulmonary fibrosis appears to be at least partially caused by an increased nuclear import of the phosphomimetic RAGE relative to wild-type RAGE. Thus, without wanting to be bound to theory, also other means that target the wild-type RAGE to the nucleus will cause an enhanced DNA repair and consequently improved remission of fibrosis.

Therefore, according to a second aspect, the invention provides a fusion protein of a RAGE protein comprising an amino acid sequence that is at least 90 % identical to a native mammalian RAGE isoform or fragment thereof, fused to at least one nuclear targeting structure.

The addition of the nuclear targeting structure also prevents the increase in membrane bound RAGE and therefore avoids or at least reduces pathological events associated with the up-regulation of RAGE.

The RAGE protein of the fusion protein may for example be derived from murine RAGE. In this case the amino acid sequence is at least 95 % identical to SEQ ID NO: 1 or a fragment thereof. Preferably, the RAGE is at least 98 % identical, more preferably at least 99 % identical to murine RAGE identified by SEQ ID NO: 1 or a fragment thereof. The fragment of murine RAGE may for example contain of amino acids 40 to 214 of SEQ ID NO: According to one embodiment the fragment of murine RAGE contains amino acids 23 to 221 of SEQ ID NO: 1. According to one embodiment the fragment of murine RAGE contains amino acids 23 to 317 of SEQ ID NO: 1. According to one embodiment the fragment of murine RAGE contains amino acids 23 to 404 of SEQ ID NO: 1.

The RAGE protein in the fusion protein according to the second aspect may be a phosphomimetic RAGE protein according to the first aspect. Even though the phosphomimetic RAGE protein is shown to localize to the nucleus, an additional NLS may increase the efficiency of the nuclear localization of the phosphomimetic RAGE protein, thereby enhancing the DNA repair and reducing inflammatory side effects of the membrane bound RAGE protein.

A nuclear targeting structure according to the invention may any moitey or sequence that with the potential of delivering the RAGE protein to the nucleus. The nuclear targeting structure is in particular a nuclear localization sequences (NLS). Typically, an NLS consists of one or more short sequences of positively charged lysines or arginines exposed on the protein surface, but other types of NLS are known. Non-limiting examples of NLSs include an NLS sequence derived from: the NLS of the SV40 virus large T-antigen, having the amino acid sequence PKKKRKV (SEQ ID NO: 7); the NLS from nucleoplasmin (e.g. the nucleoplasmin bipartite NLS with the sequence KRPAATKKAGQAKKKK (SEQ ID NO: 8); the c-myc NLS having the amino acid sequence PAAKRVKLD (SEQ ID NO: 9) or RQRRNELKRSP (SEQ ID NO: 10); the hRNPA1 M9 NLS having the sequence NQSSNFGPMKGGNFGGRSSGPYGGGGQYFAKPRNQGGY (SEQ ID NO: 11); the sequence RMRIZFKNKGKDTAELRRRRVEVSVELRKAKKDEQILKRRNV (SEQ ID NO: 12) of the IBB domain from importin-alpha; the sequences VSRKRPRP (SEQ ID NO: 13) and PPKKARED (SEQ ID NO: 14) of the myoma T protein; the sequence PQPKKKPL (SEQ ID NO: 15) of human p53; the sequence SALIKKKKKMAP (SEQ ID NO: 16) of mouse c-abl IV; the sequences DRLRR (SEQ ID NO: 17) and PKQKKRK (SEQ ID NO: 18) of the influenza virus NS1; the sequence RKLKKKIKKL (SEQ ID NO: 19) of the Hepatitis virus delta antigen; the sequence REKKKFLKRR (SEQ ID NO: 20) of the mouse Mx1 protein; the sequence KRKGDEVDGVDEVAKKKSKK (SEQ ID NO: 21) of the human poly(ADP-ribose) polymerase; and the sequence RKCLQAGMNLEARKTKK (SEQ ID NO: 22) of the steroid hormone receptors (human) glucocorticoid.

When more than one NLS is present, each may be selected independently of the others, such that a single NLS may be present in more than one copy and/or in combination with one or more other NLSs present in one or more copies. The nuclear targeting structure may be fused to the C-terminus or the N-terminus of the RAGE protein. Preferably, the NLS is linked to the N-terminus.

The fusion protein may be linked to one or more affinity tags. Examples of affinity tags are polyhistidine, protein A, glutathione S transferase, substance P, FLAG, streptavidin, and an immunoglobulin heavy chain constant region.

## Polynucleotide

According to a third aspect, the invention provides an isolated polynucleotide with a nucleic acid sequence encoding the phosphomimetic RAGE according to the first aspect or the fusion protein according to the second aspect.

The techniques used to isolate or clone a polynucleotide encoding a peptide are known in the art and include isolation from genomic DNA, preparation from cDNA, or a combination thereof. The cloning of the polynucleotides from such genomic DNA can be effected, e.g., by using the well-known polymerase chain reaction (PCR) or antibody screening of expression libraries to detect cloned DNA fragments with shared structural features. See, e.g., Innis et al., 1990, PCR: A Guide to Methods and Application, Academic Press, New York. Other nucleic acid amplification procedures such as ligase chain reaction (LCR), ligation activated transcription (LAT) and polynucleotide-based amplification (NASBA) may be used.

According to one embodiment, isolated polynucleotide according comprises a nucleic acid sequence that is at least 95 % identical, preferably at least 98 % identical, more preferably at least 99 % identical to the SEQ ID NO: 5, encoding murine phosphomimetic RAGE.

According to an alternative embodiment, isolated polynucleotide according comprises a nucleic acid sequence that is at least 95 % identical, preferably at least 98 % identical, more preferably at least 99 %, most preferably 100 % identical identical to the SEQ ID NO: 6, encoding human phosphomimetic RAGE.

This isolated polynucleotide is in particularly useful for insertion into an expression vector for *in vitro* expression of the RAGE protein according to the first aspect or the RAGE fusion protein according to the second aspect. Alternatively, the isolated polynucleotide may be used for *in vivo* expression of the RAGE protein, i.e. gene therapy.

## Vector for gene transfer or in vitro expression

In a fourth aspect, the invention also relates to an expression vector comprising a polynucleotide according to the third aspect. For gene therapy, the isolated polynucleotide is in particular introduced into a vector, either for transient transfection and expression of the protein in the patient's cells or for stable integration into the genome of the patients cells introduced. The vector may be transgene expression vector to be used for gene therapy, in particular *in vivo* expression of the phosphomimetic RAGE protein according to the first or the fusion protein according to the second aspect.

The transgene expression vector may be for example a virus derived vector such as a lentivirus vector, an adenovirus vector or an adeno-associated virus (AAV) vector. In particular the transgene expression vector may a recombinant adeno-associated virus (rAAV) vector.

The adeno-associated virus AAV belongs to the genus Dependo virus, which in turn belongs to the subfamily of the Parvovirinae, also referred to as parvoviruses, which are capable of infecting vertabrates. Parvovirinae belong to family of small DNA animal viruses, i.e. the Parvoviridae family. As may be deduced from the name of their genus, members of the Dependovirus are unique in that they usually require coinfection with a helper virus such as adenovirus or herpes virus for productive infection in cell culture. The genus Dependovirus includes AAV, which normally infects humans, and related viruses that infect other warm-blooded animals (e.g., bovine, canine, equine, and ovine adeno-associated viruses). Further information on parvoviruses and other members of the Parvoviridae is described in Kenneth I. Berns, "Parvoviridae: The Viruses and Their Replication," Chapter 69 in Fields Virology (3d Ed. 1996)

A rAAV is derived from the wild type adeno-associated virus (wtAAV) by using molecular methods. The rAAV vector is distinguished from a wtAAV vector, since all or a part of the viral genome has been replaced with a transgene, which is a non-native nucleic acid with respect to the AAV nucleic acid sequence as further defined herein.

The transgene expression vector further preferably comprises control elements such as a promoter, and transcriptional and translational stop signals. In order to be able to provide expression of the phosphomimetic RAGE in the diseased cells or tissue, the promoter may be a tissue specific promoter. Preferably the promoter is selected from the group consisting of a keratinocyte specific promoter, a liver specific promoter, a fibroblast specific promoter or a macrophage specific promoter.

The rAAV virus carrying the gene encoding phosphomimetic RAGE or the fusion protein comprising RAGE may be produced as described in Example 1.

Alternatively, the expression vector may be used for *in vitro* expression of the phosphomimetic RAGE protein according to the first or the fusion protein according to the second aspect.

The expression vector further preferably comprises control elements such as a promoter, and transcriptional and translational stop signals. The polynucleotide of according to the third aspect and of the control elements may be joined together to produce a recombinant expression vector that may include one or more restriction sites to allow for insertion or substitution of the polynucleotide encoding the polypeptide at such sites. The polynucleotide may be inserted into an appropriate expression vector for expression. In creating the expression vector, the coding sequence is located in the expression vector so that the coding sequence is operably linked with the appropriate control sequences for expression.

The *in vitro* expression vector may be any vector (e.g., a plasmid or virus) that can be conveniently subjected to recombinant DNA procedures and can bring about expression of the polynucleotide of the fourth aspect of the invention. The choice of the *in vitro* expression vector will typically depend on the compatibility of the expression vector with the host cell into which the expression vector is to be introduced. The expression vectors may be a linear or closed circular plasmid.

The *in vitro* expression vector may be adapted for cell-based or cell-free expression. The expression vector may be an autonomously replicating vector, i.e., a vector that exists as an extrachromosomal entity, the replication of which is independent of chromosomal replication, e.g., a plasmid, an extrachromosomal element, a minichromosome, or an artificial chromosome.

For autonomous replication, the vector may further comprise an origin of replication enabling the vector to replicate autonomously in the host cell in question. The origin of replication may be any plasmid replicator mediating autonomous replication that functions in a cell. The term "origin of replication" or "plasmid replicator" means a polynucleotide that enables a plasmid or vector to replicate in vivo.

Alternatively, the vector may be one that, when introduced into the host cell, is integrated into the genome and replicated together with the chromosome(s) into which it has been integrated. For integration into the host cell genome, the expression vector may rely on any other element of the expression vector for integration into the genome by homologous or non-homologous recombination. Alternatively, the vector may contain additional polynucleotides for directing integration by homologous recombination into the genome of the host cell at a precise location in the chromosome.

The in vitro expression vectors of the present invention preferably contain one or more (e.g., several) selectable markers that permit easy selection of transformed, transfected, transduced, or the like, cells. A selectable marker is a gene the product of which provides for biocide or viral resistance, resistance to heavy metals, prototrophy to auxotrophs, and the like.

The procedures used to ligate the elements described above to construct the recombinant expression vectors of the present invention are well known to one skilled in the art (see, e.g., Sambrook et al, 1989, supra).

**Method of treatment /composition for use**

According to a fifth aspect the invention provides a composition comprising carrier and an active pharmaceutical ingredient, selected from a phosphomimetic RAGE protein according to the first aspect, a fusion protein according to the second aspect and a vector according to the fourth aspect, for use in the treatment or prevention of a disease in a patient. The subject matter of the fifth aspect can also be defined as a method for the treatment or prevention of a disease in a patient, comprising administering to the patient a composition comprising carrier and an active pharmaceutical ingredient, selected from a phosphomimetic RAGE protein according to the first aspect, a fusion protein according to the second aspect and a vector according to the fourth aspect.

According to one embodiment of the fifth aspect a composition for use in the treatment or prevention of a disease in a patient, is provided wherein the composition comprises a carrier and a a vector according to the fourth aspect according to the first aspect. Instead of administering the gene in form of a vector it may also be possible to transfect or transduce the isolated polynucleotide. Thus, according to an alternative embodiment of the fifth aspect a composition for use in the treatment or prevention of a disease in a patient, is provided wherein the composition comprises a carrier and the isolated polynucleotide according to the third aspect. According to an alternative embodiment of the fifth aspect a composition for use in the treatment or prevention of a disease in a patient, is provided wherein the composition comprises a carrier and a phosphomimetic RAGE protein according to the first aspect. According to an alternative embodiment of the fifth aspect a composition for use in the treatment or prevention of a disease in a patient, is provided wherein the composition comprises a carrier and a a fusion protein according to the second aspect.

As shown in the examples, administration of the phosphomimetic RAGE in form of a vector to diabetic mice with pulmonary fibrosis leads to a remission of the fibroses and a recovery of the pulmonary function to the level of a healthy individual. Accordingly the *in vivo* expressed phosphomimetic RAGE successfully enhances DNA repair such that pulmonary fibrosis is not only stopped but reduced. However, the effect of the phosphomimetic RAGE is not limited to lung tissue. The inventors were able to show that phosphomimetic RAGE reduces renal fibroses and DNA damage in mice with induced diabetes (see Example 7 and Fig. 6). Moreover, the transduction of AAV2/8-RAGE$^{S376E-S389E}$ virions improves survival rate in reperfusion injury of diabetic mice kidney. Finally, The phosphomimetic mutant of RAGE improves the nerve regeneration. (see Example 6 and Figure 5).

Based on this evidence, it can be reasonably expected that the phosphomimetic RAGE has an effect on any disease that involves DNA damages such as diseases initiated by impaired DNA repair, diseases initiated by increased DNA damage or diseases initiated by increased senescence.

According to one embodiment, the disease is a disease of the lung, nerve, kidney, blood vessels or lymphatic vessels. Preferably, the disease is a disease of the lung, nerve or kidney.

According to one embodiment, the disease is selected from age associated diseases, liver fibrosis, diabetic complications, neurodegenerative diseases, radiation damage, ischemia reperfusion, stroke, myocardial infarction, tissue damage associated with renal failure and replacement therapy, septicemia, skin disorders such as impaired wound healing and psoriasis, pulmonary fibrosis, wherein the pulmonary fibrosis is in particular caused by smoking, pollution, diabetes, radiation, or chemotherapy.

Suitable carriers for introducing the vector or the phosphomimetic RAGE include viral carriers and non-viral carriers. Examples of viral carriers are adenovirus capsids, adeno associated virus capsids, lentivirus capsids and virus like particles (VLP) such as VLP from parvoviridae.

Viral systems such adenovirus, AAV, or lentivirus may provide both the (expression) vector and the capsid as carrier.

Non-viral carriers are for example PEI nanoparticles, liposome or gelatin nanoparticles, liposomes, immunoglobulins or fragments thereof. The PEI nanoparticles are formed by the polymer polyetherimide (PEI), have a diameter of 50-100 nm and are considered the long-term safe and biocompatibility. Gelatin is a protein derived from collagen. Cationic gelatin nanoparticles are 100-300 nm in diameter and are produced by chemically introducing cations such as thylenediamine, putrescine, spermidine, or spermine to the carboxyl group of gelatin. Liposomes are sphere-shaped vesicles consisting of one or more phospholipid bilayers. Immunoglobulins or fragments thereof include full antibodies, Fab fragments, $F(ab')_2$ fragments, Fd fragments, Fv fragments, dAb fragments, isolated complementarity determining region (CDR) and an single chain Fv (scFv).

According to preferred embodiment, the API is a rAAV vector containing a polynucleotide encoding a phosphomimetic RAGE protein and the carrier is the an AAV capsid. Preferably the capsid is specific for the diseased tissue or cells. According to one embodiment the AAV system (rAAV vector/capsid) is AAV2/8. Different AAV capsids are specific for different tissues. Thus, the AAV capsid should be selected based on the disease to be treated.

In order to increase the specificity, the composition may further comprise a cell targeting moiety that specifically targets the diseased cells of the patient. The cell targeting moiety may be for example a peptide specifically bound by a tissue specific cell receptor.

Compositions of the embodiments can be prepared and administered to a subject by any methods well known in the art of pharmacy. See, e. g, Goodman & Gilman's The Pharmacological Basis of Therapeutics, Hardman et al., eds., McGraw-Hill Professional (10th ed., 2001); Remington: The Science and Practice of Pharmacy, Gennaro, ed., Lippincott Williams & Wilkins (20th ed., 2003); and Pharmaceutical Dosage Forms and Drug Delivery Systems, Ansel et al. (eds), Lippincott Williams & Wilkins (7th ed., 1999). In addition, the pharmaceutical compositions of the embodiments may also be formulated to include other medically useful drugs or biological agents.

The compositions can be administered to the patient by any customary administration route, e. g., orally, parenterally, or by topic application by inhalation. Parenteral administration includes intravenous injection, subcutaneous injection, intraperitoneal injection, intramuscular injection, liquid agents, suspensions, emulsions and dripping agents. For parenteral administration the pharmaceutical composition should be an injectable agent such as a liquid agent or a suspension. For this, the composition is preferably fourmulated in a carrier liquds such as sterile pyrogen-free water and sterile pyrogen-free saline solution. Other potential components of the formulation binders, disintegrants, surfactants, absorption accelerators, moisture retention agents, absorbers, lubricants, fillers, extenders, moisture imparting agents, preservatives, stabilizers, emulsifiers, solubilising agents, salts which control osmotic pressure, diluting agents such as buffers and excipients usually used depending on the use form of the formulation. These are optionally selected and used depending on the unit dosage of the resulting formulation. According to one embodiment, the use comprises an administering the composition to the patient intravenously (IV), intraperitoneally (IP), by inhalation or by topic application.

The composition of a rAAV containing a polynucleotide according to the third aspect and an AAV capsid as carrier may be transduced to the patient in a dose in the range of $10^{12}$ to $10^{16}$ virions/kg of body weight in the primary infection. Preferably the dose in the primary rate is in the range of $10^{13}$ to $10^{15}$ virions/kg of body weight, more preferably 8 $10^{13}$ to $10^{14}$ virions/kg of body weight

After initial application further booster infections may be applied. The dosage in a booster infection may be in the range of $10^{11}$ to $10^{15}$ virions/kg of body weight in the primary infection. Preferably the dose in the primary rate is in the range of $10^{12}$ to $10^{14}$ virions/kg of body weight, more preferably 6 $10^{12}$ to $10^{13}$ virions/kg of body weight

The composition may also be used in cell therapy. Thus according to one embodiment of the fifth aspect, the active pharmaceutical ingredient is a vector according fourth aspect, and the treatment is a cellular therapy comprising the steps of:

- obtaining cells from a diseased tissue of a patient;
- culturing the cells;
- transfecting the cells of the patient with the composition, and
- aministering the transfected cells from the patient.

**Production Method**

According to a sixth aspect, the invention provides a process of producing the phosphomimetic RAGE protein according to the first aspect or fusion protein according to the second aspect of the invention. The process at least comprises the steps of protein expression purification of the protein.

For expression of fusion protein cell-based or cell-free (*in vitro*) expression systems may be used. Common cell based systems are bacteria, such as *E.coli*, *B. subtilis,* yeast, such as *S. cerevisiae* or eukaryotic cell lines, such as baculovirus infected Sf9 cells mammalian cells like CHO cells, HEK293 cells or HeLa cells.

Thus, according to one embodiment of the sixth aspect of the invention the process of producing the phosphomimetic RAGE or the fusion protein according to the first and second aspect of the invention comprises at least the steps of:

a) introducing a polynucleotide according to third aspect into a host cell;
b) culturing the transformed host cell in a medium under conditions leading to a protein expression with the polynucleotide as a template;
c) isolating the expression product.

The expression vector according to the fourth aspect is introduced into a host cell so that the expression vector is maintained as a chromosomal integrant or as a self-replicating extrachromosomal vector as described earlier.

The term "host cell" encompasses any progeny of a parent cell that is not identical to the parent cell due to mutations that occur during replication. The choice of a host cell will, to a large extent, depend upon the gene encoding the polypeptide and its source. The host cell may be any cell useful in the recombinant production of a polypeptide of the present invention, e.g., a prokaryote or a eukaryote.

The prokaryotic host cell may be any Gram positive bacterium or a Gram negative bacterium. Gram positive bacteria include, but not limited to, Bacillus, Streptococcus, Streptomyces, Staphylococcus, Enterococcus, Lactobacillus, Lactococcus, Clostridium, Geobacillus, and Oceanobacillus. Gram negative bacteria include, but not limited to, *E.coli,* Pseudomonas, Salmonella, Campylobacter, Helicobacter, Flavobacterium, Fusobacterium, Ilyobacter, Neisseria, and Ureaplasma. The host cell may also be a eukaryote, such as a mammalian, insect, plant, or fungal cell. Preferably, the host cell is *E.coli.*

Methods of cultivation of host cells in a nutrient medium suitable for production of the fusion protein are well known in the art. For example, the host cell may be cultivated by shake flask cultivation, small-scale or large-scale fermentation (including continuous, batch, fed-batch, or solid state fermentations) in laboratory or industrial fermentors performed in a suitable medium and under conditions allowing the fusion protein to be expressed. The cultivation takes place in a suitable nutrient medium comprising carbon and nitrogen sources and inorganic salts, using procedures known in the art. Suitable media are available from commercial suppliers or may be prepared according to published compositions (e.g., in catalogues of the American Type Culture Collection).

Depending on the host/vector system used, the fusion protein may or may not be secreted into the nutrient medium. In case it is secreted, the polypeptide can be recovered directly from the medium. Otherwise, the cells are separated from the culture medium and lysed.

Methods of cell lysis are known in the art. Non-limiting examples of the methods of cell lysis are mechanical disruption, liquid homogenization, sonication, freeze-thaw procedure or mortar and pestle.

The phosphomimetic RAGE protein or fusion protein may be recovered using methods known in the art. For example, the phosphomimetic RAGE protein or fusion protein may be recovered from the nutrient medium by conventional procedures including, but not limited to, centrifugation, filtration, extraction, spray-drying, evaporation, or precipitation.

The phosphomimetic RAGE protein or fusion protein may be purified by a variety of procedures known in the art including, but not limited to, chromatography (e.g., ion exchange, affinity, hydrophobic, chromatofocusing, and size exclusion), electrophoretic procedures (e.g., preparative isoelectric focusing), differential solubility (e.g., ammonium sulfate precipitation), SDS-PAGE, or extraction (see, e.g., Protein Purification, J.-C. Janson and Lars Ryden, editors, VCH Publishers, New York, 1989) to obtain substantially pure polypeptides.

## Method of purification of RAGE in high purity

According to the present state of the art, full length RAGE is generally purified using a polyhistidine tag affinity chromatography, in particular polyhistidine Ni-NTA purification system the Qiaexpressionist® method commercialized by Qiagen.

A polyhistidine-tag is an amino acid motif in proteins that consists of at least six histidine (His) residues, often at the N- or C-terminus of the protein. It is also known as hexa histidine-tag, 6xHis-tag, His6 tag and by the trademarked name His-tag (registered by EMD Biosciences).

Polyhistidine-tags bind to chelators, such as iminodiacetic acid (Ni-IDA) and nitrilotriacetic acid (Ni-NTA) for nickel and carboxylmethylaspartate (Co-CMA) for cobalt, with micromolar affinity. Suitable resins for purification are sepharose/agarose functionalised with the chelator.

However, as shown in Example 8, while this purification method achieves a certain degree of purity of the RAGE protein, the purity is not sufficient for a medical use of the full-length RAGE protein. The final purification samples of all tested RAGE proteins, but in particular the RAGE mutant proteins contained several protein in addition to the purified RAGE. In this regard it is referred to Fig .7 showing the results of an SDS-PAGE analysis. In the gel, in addition to the RAGE protein band at 55 kDA both higher molecular and lower molecular protein bands are invisible.

The inventors have defined a novel purification method based on a first purification step, in particular poly-histidine Ni-NTA chromatography, in combination with heparin chromatography. This purification method provides highly purified RAGE proteins, both wild-type and mutant RAGE.

Immobilized heparin has two main modes of interaction with proteins. It is known have an affinity for a wide range of biomolecules, including coagulation factors and other plasma proteins, lipoproteins, protein synthesis factors, enzymes that act on nucleic acids, and steroid receptors. Heparin also functions as a high capacity cation exchanger, due to its anionic sulfate groups. However, no binding off RAGE to heparin has been described so far.

Thus, according to a seventh aspect the invention provides a method of purification of a RAGE protein with a polyhistidine-tag, comprising the steps of:

- Providing a first solution comprising the RAGE protein with a polyhistidine-tag and contaminant proteins;

- Subjecting the first solution to a Ni-NTA chromatography and eluting a second solution containing the RAGE protein; and
- Subjecting the second solution to a heparin chromatography and eluting a third solution containing the RAGE protein;

According to one embodiment of the seventh aspect the method further comprises subjecting the third solution containing the RAGE protein to a gel filtration chromatography, in particular Sephacryl chromatography.

The first, second and third solution may be subjected to filtration and/or buffer exchange. The first solution is preferably the supernatant obtained by lysis of the host cells in which the RAGE protein is produced and the subsequent centrifugation. Lysis of the cells can be performed with any of the methods listed above. Preferably lysis is caused by sonication. The lysis buffer preferably contains a Tris-HCl buffer, NaCl, glycerol, imidazole, and beta-mercaptoethanol.

The concentration of Tris-HCl in the lysis buffer maybe in the range from 5 to 100 mM. Preferably, the concentration of Tris-HCl is in the range from 10 to 50 mM. More preferably, the concentration of Tris-HCl is in the range from 15 to 35 mM. Most preferably, the concentration of Tris-HCl is in the range from 20 to 30 mM, in particular the concentration of Tris-HCl is about 25 mM.

The concentration of NaCl in the lysis buffer maybe in the range from 50 to 2000 mM. Preferably, the concentration of NaCl is in the range from 100 to 1000 mM. More preferably, the concentration of NaCl is in the range from 300 to 800 mM. Most preferably, the concentration of NaCl is in the range from 400 to 500 mM, in particular the concentration of NaCl is about 450 mM.

The concentration of glycerol in the lysis buffer maybe in the range from 1 to 50 wt.-%. Preferably, the concentration of glycerol is in the range from 2 to 40 wt.-%. More preferably, the concentration of glycerol is in the range from 5 to 20 wt.-%. Most preferably, the concentration of glycerol is in the range from 8 to 12 wt.-%, in particular the concentration of glycerol is about 10 wt.-%.

The concentration of immidazole in the lysis buffer maybe in the range from 10 to 100 mM. Preferably, the concentration of immidazole is in the range from 15 to 90 mM. More preferably, the concentration of immidazole is in the range from 20 to 80mM. Most preferably, the concentration of immidazole is in the range from 40 to 60 mM, in particular the concentration of immidazole is about 50 mM.

The concentration of beta-mercaptoethanol in the lysis buffer maybe in the range from 0.1 to 10 mM. Preferably, the concentration of beta-mercaptoethanol is in the range from 0.5 to 8 mM. More preferably, the concentration of beta-mercaptoethanol is in the range from 1 to 5 mM. Most preferably, the concentration of beta-mercaptoethanol is in the range from 1.5 to 2.5 mM, in particular the concentration of beta-mercaptoethanol is about 2 mM.

The pH of the lysis buffer is in particular in the range from 7 to 8. Preferably, the pH of the lysis buffer is in the range from 7.2 to 7.8. More preferably, the pH of the lysis buffer is in the range from 7.4 to 7.6, in particular, the pH is about 7.5.

For polyhistidine purification any Ni-NTA resin known in the art is applicable. Preferably, the resin is a Ni-NTA Agarose from the Qiaexpressionist® system from Qiagen. The binding is preferably carried out in batch mode. The binding time may be in the range from 20 min to 10 h. Preferably the binding time is in the range from 1 h to 5 h. More preferably, the binding time is in the range from 2.5 to 3.5 h.

After binding of the first solution to the Ni-NTA column, the flow through is removed and the column is washed, preferably with the lysis buffer. The bound RAGE protein is then eluted with an Ni-NTA elution buffer. The Ni-NTA elution buffer preferably contains the same components as the lysis buffer but an increased concentration of imidazole.

The concentration of immidazole in the lysis buffer maybe in the range from 100 to 1000 mM. Preferably, the concentration of immidazole is in the range from 200 to 800 mM. More preferably, the concentration of immidazole is in the range from 400 to 600 mM. Most preferably, the concentration of immidazole is in the range from 450 to 550 mM, in particular the concentration of immidazole is about 500 mM.

The elution may be carried out using a gradient increasing the concentration of imidazole. After elution of the second solution from the Ni-NTA column the buffer may be exchanged, preferably by dialysis. The buffer exchanged second solution may in particular contain Tris-Cl, KCl and beta-mercaptoethanol.

The concentration of Tris-HCl in the buffer exchanged second solution maybe in the range from 5 to 100 mM. Preferably, the concentration of Tris-HCl is in the range from 10 to 50 mM. More preferably, the concentration of Tris-HCl is in the range from 15 to 35 mM. Most preferably, the concentration of Tris-HCl is in the range from 20 to 30 mM, in particular the concentration of Tris-HCl is about 25 mM.

The concentration of KCl in the buffer exchanged second solution maybe in the range from 10 to 500 mM. Preferably, the concentration of KCl is in the range from 20 to 200 mM. More preferably, the concentration of KCl is in the range from 50 to 150 mM. Most preferably, the concentration of KCl is in the range from 80 to 120 mM, in particular the concentration of KCl is about 100 mM.

The pH of in the buffer exchanged second solution may be in the range from 7 to 8. Preferably, the pH of the lysis buffer is in the range from 7.2 to 7.8. More preferably, the pH is in the range from 7.4 to 7.6, in particular, the pH is about 7.5.

For heparin chromatography, the resin may be any heparin resin known in the art. The heparin resin is preferably Heparin Sepharose 6 Fast Flow® (GE Healthcare Life Sciences). The binding is preferably carried out in batch mode. The binding time may be in the range from 20 min to 100 min. Preferably the binding time is in the range from 40 min to 80 min. More preferably, the binding time is in the range from 50 to 70 min.

The third solution eluted from the heparin resin using a heparin elution buffer. The heparin elution buffer may in particular contain Tris-Cl, NaCl and beta-mercaptoethanol.

The concentration of Tris-HCl in the buffer exchanged second solution maybe in the range from 5 to 100 mM. Preferably, the concentration of Tris-HCl is in the range from 10 to 50 mM. More preferably, the concentration of Tris-HCl is in the range from 15 to 35 mM. Most preferably, the concentration of Tris-HCl is in the range from 20 to 30 mM, in particular the concentration of Tris-HCl is about 25 mM.

The concentration of NaCl in the heparin elution buffer maybe in the range from 50 to 2000 mM. Preferably, the concentration of NaCl is in the range from 100 to 1000 mM. More preferably, the concentration of NaCl is in the range from 300 to 800 mM. Most preferably, the concentration of NaCl is in the range from 400 to 600 mM, in particular the concentration of NaCl is about 500 mM.

The pH of in the heparin elution buffer may be for example be in the range from 7 to 8. Preferably, the pH of the lysis buffer is in the range from 7.2 to 7.8. More preferably, the pH is in the range from 7.4 to 7.6, in particular, the pH is about 7.5.

The invention is further explained by the following illustrative examples.

## EXAMPLES

### Example 1: Virus production

The production of recombinant AAV virions in HEK293 cells was performed as described in O'Driscoll (2012). Cells were seeded at 5 x $10^6$ cells/flask for each vector type. Once the cells reached 80% confluency, theywere transfected with the respective plasmids presented in Table 1. The triple-transfection of HEK 293T cells was set up as follow: for each confluent T150 flask, 12.5 μg of AAV backbone plasmid (cloned into pAM CBA WPRE bGH backbone plasmid by replacing GFP with the RAGE-RFP expression cassette; Table 1), 25 μg DP2rs helper plasmid and 12 μg pXr8 plasmid were added to 2.4 mL of sterile water in a 15 mL reaction tube, then 330 μL of 2.5 M $CaCl_2$ was added to the mixture. The transfection mixture was filtered through a 0.2 μm syringe filter into another 15 mL reaction tube and whilst mixing the solution vigorously, 2.5 mL of 2x HeBs buffer (280 mM NaCl, 1.5 mM $Na_2HPO_4$, 50 mM HEPES, pH 7.5) was added. The mixture was incubated 5 min at room temperature and 5 mL was applied to the corresponding T150 flask.

Transfected cells were incubated at 37°C/5% CO2. 16 h post-transfection, media was removed and replaced with fresh complete DMDM. 96 h post-transfection, cells were lysed in lysis buffer (50 mM Tris, 150 mM NaCl at pH 8.4) by vigorous mixing to release the AAV virions. Lysate and culture medium samples were incubated at 37°C for 30 min in a water bath. cell debris was removed by centrifugation at 3000 g for 15 min at 4°C. Virions were purified and concentrated using an lodixanol gradient and a Vivaspin centrifugal concentrator (50 KDa cutoff).

**Table. 1: Details of plasmids used for AAV production.**

| AAV construct | Plasmid |
|---|---|
| RAGE-RFP $^{WT}$ | pAM CBA RAGERFP WPRE bGH & pDP2rs + Xr8. |
| RAGE-RFP $^{S376A-S389A}$ | pAM CBA RAGE mut RFP WPRE bGH & pDP2rs + Xr8. |
| RAGE-RFP $^{S376E-S389E}$ | pAM CBA RAGE mut RFP WPRE bGH & pDP2rs + Xr8. |
| RFP control | pAM CBA RFP WPRE bGH & pDP2rs + Xr8. |

### Example 2: Measuring the pulmonary lung function and static compliance of diabetics can be improved by AAV2/8-RAGE$^{S376E-S389E}$ virions.

In order to test the ability of phosphomimetic RAGE to restore lung functionality and static compliance of diabetics, streptozotocin induced diabetic mice (six month of diabetes age) were transduced with AAV2/8-RAGE$^{S376E-S389E}$ virions.

Mice used

Two different mouse strains were used in this study. The wild type (WT) and RAGE knock out (RAGE$^{-/-}$) mice were in the same C57BL6 background. The $RAGE^{-/-}$ mouse was created as described in Constein, R., *et al.* (2001). WT mice were obtained from Charles River, Boston USA. The mice were housed in groups of four and a 12h/12h light/dark cycle with food and water *ad libitum.* Diabetes was induced by i. p. administration of STZ at (60mg/kg), freshly dissolved in sterile sodium citrate (0.05M, pH4.5) on 6 subsequent days. Mice serving as controls were administered sodium citrate only. Diabetes was verified 16-25 days post-administration by measurement of blood glucose levels in samples from the tail vein. In the first two weeks following diabetes induction, blood glucose was measured daily. If glucose levels occasionally recovered, an additional STZ injection was given on day 25-27. More than 90% of mice became diabetic within the first four weeks. As soon as blood glucose increased above 300mg/dl, individual supplementation with 1-2U Insulin Glargine (40U/ml) was started. Once glucose levels had stabilized between 400 mg/dl and 450 mg/dl, blood glucose was determined at least once a week for the first two months, and thereafter every second week. Tight blood glucose control kept individual blood glucose levels stable throughout the experiments (healthy controls: 136.2 $\pm$ 3.06 mg/dl diabetic mice: 408.3 $\pm$ 15.3 mg/dl; p <0.0001). At the end of the experiments functional assays were performed, before the animals were sacrificed with $CO_2$ and organs were processed according to the respective assay.

Transduction of mice

Viral inoculums for transduction were prepared in saline. Isofluorene anaesthetized mice were then hanged on the angled wooden platform (hanging by its incisors on the surgical thread) and gently restrained it in place with a piece of ribbon and gently open its mouth to pull its tongue out with forceps and deliver the viral load directly into the trachea slowly along the side of the wall of trachea without blocking the access of air. This procedure was carried out gently so that no tissue damages occurred. The mouse was held upright for a few seconds to allow the inoculum to be inhaled into the lungs.

Later mice were rested in normal growth conditions as described above for 4 more weeks and monitored regularly.

Lung function measurement

In order to evaluate lung mechanics, invasive lung function analysis was performed as described in Wielputz *et al.* (2011). For this purpose, mice were anesthetized with sodium pentobarbital (80 mg/kg), tracheostomized, and placed on a small animal ventilator (FlexiVent system, SCIREQ, Montreal, QC, Canada). To prevent spontaneous breathing, mice were then paralyzed with pancuronium bromide (0.5 mg/kg) and ventilated with a tidal volume of 10 mL/kg at a frequency of (150 breaths/min) and a positive end expiratory pressure of 3cm $H_2O$ to prevent alveolar collapse. Pressure-volume curves with stepwise increasing pressure (PVs-P) were consecutively measured. All perturbations were performed until three acceptable measurements were achieved.

As can be seen in Figure 1, diabetic mice transduced with the phosphomimetic RAGE mutant showed increased lung function and improved static compliance compared to the saline treated negative control. Diabetic mice treated with saline only exhibit a severe reduced pulmonary function compared to non-diabetic mice. This is in line with the fact that diabetes leads to emerging fibrosis within lung tissue. As a consequence, the lung loses is dynamics resulting in a decreased function. Mice transduced with AAV2/8-$RAGE^{S376A-S389A}$ virions exhibit a slightly improved lung function compared to the saline treated controls. Even better pulmonary function was observed in mice transduced with murine $RAGE^{WT}$. However, significantly improved lung function and static compliance was achieved when mice were transduced with the phosphomimetic RAGE mutant (AAV2/8-$RAGE^{S376E-S389E}$) that performed in principle in same levels as non-diabetic mice.

**Example 3: Transduction of AAV2/8-$RAGE^{S376E-S389E}$ virions timely improves the efficiency of survival in reperfusion injury of diabetic mice kidney.**

As phosphomimetic RAGE could improve the lung function of diabetic mice a possible function of the RAGE mutant was tested in mice suffering from a kidney injury. WT mice were treated with Streptozotocin in order to induce diabetes (8-10 mice per group). Next, mice diabetic for 6 months were pretreated with AAV2/8-$RAGE^{S376E-S389E}$ virions for four weeks. Reperfusion injury was induced as described in Dessing *et al.* (2012). Data represent group averages (8-10 mice per group). To induce kidney injury, both renal arteries were clamped for 30 min under general anesthesia inhalation anesthesia with isoflurane (Baxter, Deerfield, USA) in WT non-diabetic mice (C57BL/6; 9-10 weeks) control or STZ treated mice,. Mice received a subcutaneous injection of 0.01mg/100g Buprenorphine (Temgesic, RB Pharmaceuticals, Berkshire, UK). Mice were monitored for survival for days. As a reference, mice underwent similar pre-treatment without clamping of the renal arteries.

Pre-treatment with the phosphomimetic mutant of RAGE clearly improves the survival ratio in mice suffering from reperfusion injury of the kidney, whereas mice pretreated with saline or the empty virions exhibited a reduced survival (25% lethality) under the same conditions.

**Example 4: Activated ATM-mediated phosphorylation of RAGE is essential for nuclear translocation as well as for its DNA repair function.**

Assessing RAGE phosphorylation

Kumar (2016) has shown that murine RAGE is phosphorylated by ATM. To asses if human RAGE is also a target of ATM the status of RAGE phosphorylation after DNA DSB was analyzed *in vitro* by transfecting human lung adenocarcinoma cells (A549*)*. For this purpose, following treatment with either camptothecin (CPT; 1μM) or bleomycin (BL; 30ug/ml) for 1 h, cell extracts were prepared in by resuspending the cells in 20mM Tris-Cl pH 7.5, 40mM NaCl, 2mM $MgCl_2$, 0.5% NP40, 50U/ml benzonase, supplemented with protease and phosphatase inhibitors.After 15 min of incubation on ice, the NaCl concentration was adjusted to 150/450 mM and then it was further incubated for 15 more minutes. The lysate was then centrifuged at 14K for 15 min at 4°C. The supernatant was collected in labeled tubes. hRAGE was then pulled down via the 3 FLAG-tag trapping beads (M2-agarose) and analyzed by western blotting for phosphorylation using the anti-pS/TQ antibody, which is specific for the conserved motif for DNA-PK or ATM substrates (#9607, Cell Signaling).

It was found that RAGE is phosphorylated following induction of DSBs by Bleomycin and CPT (see Figure 3 A). To identify the source of this phosphorylation, the experiment was repeated in presence of either Nu7026, a known inhibitor of DNA-PK, or Ku55933, a specific inhibitor of ATM. It was found that phosphorylation of RAGE, in response to DNA damage, is inhibited by Ku55933 but not Nu7026, suggesting that RAGE serves as a downstream target for ATM sensor kinase (see Figure 3 A).

Fluorescent microscopy

A549 cells were grown on poly L-lysine coated coverslips (Thermo) or glass bottom dishes (Ibidi) were pre-sensitized with BrdU (10μM for 24 hours in phenol red free DMEM medium) and then treated with 1μM camptothecin for 60 minutes. After incubation, cells were fixed with 4% paraformaldehyde for 15mins at 4°C and permeabilized with 0.3% Triton X-100 in PBS for 5 mins at room temperature. Samples were then blocked in 5% bovine serum albumin and immune-stained using indicated primary antibodies and secondary antibodies. Species adsorbed AlexaFluor 488/555/647 secondary antibodies were purchased from Abcam. Fluorescent images of infected and control cells were captured with a CCD camera connected to an inverted fluorescence microscope (Cell Observer, Carl Zeiss, GmbH, Göttingen, Germany). Samples were scanned using an x63 oil objective. Images were further processed using ImageJ (Fiji) and Photoshop CS5 (Adobe).

It was observed that upon induction of DNA DSB RAGE translocates to the nucleus as can be seen from Figure 3B. Initiation of DNA repair is illustrated by the co-staining with the DNA DSB repair marker γH2AX. Given the fact, that upon induction of DNA DSB RGAE becomes phosphorylated it is assumed if RAGE phosphorylation precedes its translocation into the nucleus.

**Example 5: Diabetes mediated reduced RAGE S376 and S389 phosphorylation affects the DNA repair function**

To test the phosphorylation status of RAGE in diabetic mice, western blot experiments on lung tissue extracts were performed. For this, lung tissue from diabetic mice was harvested and lysed. Mice exposed to radiation in order to generate DNA damages are compared to diabetic mice. Total body irradiation (TBI) of mice was performed with gamma irradiation using $^{137}Cs$ in a BioBeam 8000 (Gamma Service Medical GmbH, Germany) at a dose rate of 12Gy. Mice were anesthetized and held in ventilated radiation chamber for TBI. Post-radiation induced DNA damage mice were housed for another 4 hours and then sacrificed and organs were harvested. Control mice handled the same way, but were not exposed to radiation.

Total cell extracts or tissue extracts (liquid N2 grinded powder of respective tissue) were obtained by resuspending cells or tissue in 20mM Tris-Cl pH 7.5, 40mM NaCl, 2mM $MgCl_2$, 0.5% NP40, 50U/ml benzonase, supplemented with protease and phosphatase inhibitors.After 15 min of incubation on ice, the NaCl concentration was adjusted to 150/450 mM and then it was further incubated for additional 15 min. The lysate was then centrifuged at 14K for 15 min at 4°C. The supernatant was collected in labeled tubes and the total protein was quantified using Bradford's reagent. About 10 to20μg of total protein was used for loading in Laemmli buffer (0.8% SDS, 4% glycerol, 100 mM dithiothreitol, 25 mM Tris-HCl pH 6.8, 0.005% bromophenol blue). Proteins were resolved by SDS-PAGE (transferred onto nitrocellulose (Protran) and probed using the appropriate primary and secondary antibodies coupled to horseradish peroxidase (Cell Signaling or Santa Cruz). Protein detection was done via ECL reagents (GE Healthcare).

Tissue samples from diabetic mice showed elevated levels of γH2AX indicating the onset of DNA DSB repair mechanisms comparable to mice exposed to radiation. However, if compared to the irradiated tissue samples the amount of phosphorylated RAGE is significantly reduced in diabetic samples. This might be due to a reduced ATM activity with advancing diabetes age, leading to an impaired DNA DSB repair.

**Example 6: Transduction of AAV2/8-RAGE$^{S376E-S389E}$ virions timely improves deteriorating nerve function.**

The significant improvement in lungs and kidney function prompted to examine the benefits of RAGE$^{S376E-S389E}$ mutant on deteriorating nerve function of diabetic mice. To do so, mice were transduced with AAV virions and after 8-10 weeks the nerve function was analyzed. For this, WT control mice were compared to mice transduced with the respective virions. In brief, the foot withdrawal latency of the mice was measured using the Hargreaves apparatus (*Ugo Basile, Comerio, Italy*) according to the general procedures of the Hargreaves assay. The cut-off time of the equipment was preset at 33 s with an intensity of 60. Briefly, each mouse was placed into the testing enclosure and allowed time (30 min) to acclimatize before measurements were taken. Three measurements of foot withdrawal latency were made on each animal using alternating hind paws. Each trial was separated by an interval of 5min to decrease the possibly of skin injury and alteration of sensitivity of cutaneous nociceptors. As for the hot plate assay, the latency, until mice showed the first signs of discomfort, was recorded and the differences in latency times between control and treated animals determined.

**Example 7: Transduction of AAV2/8-RAGE$^{S376E-S389E}$ virions timely improves kidney function in diabetic mice**

Since the phosphomimetic RAGE mutant improves the efficiency of survival in reperfusion injury of diabetic mice kidney an analysis of the function of the RAGE mutant in renal fibrosis was conducted. Therefore, Masson's trichrome staining was performed an STZ treated kidney tissue transduced with AAV2/8-RAGE$^{S376E-S389E}$ virions. Staining was performed on tissue sections after deparaffinizing them in xylol and rehydrating them through a series of 100% ethanol, 95% ethanol, 70% ethanol then washed extensively with water and then incubated with Weigert's iron hematoxylin solution for 10 minutes and then, washed extensively with distilled water and incubated with Biebrich scarlet-acid fuchsin solution for 10-15 minutes. After incubation the slides were washed extensively and differentiated in phosphomolybdic-phosphotungstic acid solution for 10-15 minutes and transfer them to aniline blue solution and stain for 5-10 minutes. Rinse briefly in distilled water and differentiate in 1% acetic acid solution for 2-5 minutes. After this step slides were washed again with distilled water and dehydrated immediately with 95% ethyl alcohol, absolute ethyl alcohol and n-xylene. The slides were then mounted and microscopically analyzed. In Figure 6A it can be seen, that in kidney tissue of mice transduced with AAV2/8-RAGE$^{S376E-S389E}$ virions stained with Masson's trichrome staining the accumulated ECM, which forms when mice were treated with STZ, is resolved. The resolution of the ECM correlates with improved overall kidney function.

To further assess the function of the phosphomimetic RAGE in DNA damage repair on STZ induced diabetes, fluorescence microscopy on kidney sections was performed. As can be seen in Figure 6B, STZ treatment leads to an accumulation of the DNA DSB repair marker λH2AX in the nuclei. In contrast, the accumulation of λH2AX is absent in STZ treated cells transduced with AAV2/8-RAGE$^{S376E-S389E}$ virions comparable to cells of non-diabetic tissue, indicating a role of the RAGE mutant in reducing DNA damage.

**Example 8: Method for expression and purification of RAGE**

For protein expression, the pET28a-RAGE construct was transformed into *E. coli* BL21 (DE3) Codon Plus (Novagen) competent cells. The bacteria were cultured in TB medium under vigorous shaking (200 rpm) at 37 °C to a density of $OD_{600}$ = 0.8 and then transferred to 25°C. Protein expression was induced with IPTG (0.4 mM in Water). The culture was further incubated at constant conditions for 8 h. The cell culture was then centrifuged at 6000 rpm for 15 min at 4 °C. The supernatant was removed and the cell pellet was stored at -80°C.

The frozen cell pellet was resuspended in lysis buffer (50mM NaHPO4 (pH=8.0), 300mM NaCl, 10mM Imidazole) for protein purification. Celllysis was facilitated using sonication on ice bath with a pulse of 40W, while length of 2s persistent pulse was 5 m each. The recombinant protein was purified from the lysate using Ni NTA beads according to the QIAexpressionist method. The purified fractions were then analyzed onto SDS PAGE.

As can be seen in Figure 7, recombinant RAGE (55 KDa) is expressed and purified in amounts sufficient for detection using Coomassie Brilliant Blue (CBB) staining. Additional bands present in the CBB stained gel represent contaminants purified together with recombinant RAGE using the above mentioned purification method. For further analysis the purest fractions were pooled and dialyzed in 1X PBS or indicated reaction buffer overnight at 4°C.

## Example 9: Improved method for expression and purification of ultrapure RAGE

For protein expression, the pET28a-RAGE construct was transformed into *E. coli* BL21 (DE3) Codon Plus (Novagen) competent cells. The bacteria were cultured in TB medium under vigorous shaking (200 rpm) at 37 °C to a density of $OD_{600}$ = 0.6 and then transferred to 25°C. Protein expression was induced with IPTG (0.3 mM in Water). The culture was further incubated at constant conditions for 12 h. Next, the cell culture was centrifuged at 6000 rpm for 15 min at 4 °C. The supernatant was removed and the cell pellet was stored at -80°C.

The pellet was then resuspended in lysis buffer (25mM Tris-Cl (pH=7.5), 450mM NaCl, 10% Glycerol, 50mM Imidazole, 2mM β-mercaptoethanol) and sonicated on ice bath with a pulse of 40W, while length of 2s persistent pulse was 5 min each. The lysate was then clarified by centrifuging it at 12K for 45min at 4°C. The supernatant was used for purifying the recombinant protein via Ni-NTA beads in batch mode for 3h at 4°C. After binding the beads were extensively washed with lysis buffer and the protein was eluted with linear or step gradients (100mM to 500mM) of imidazole in lysis buffer. The purified protein was analyzed on SDS PAGE and pooled fractions were then extensively dialyzed in 25mM Tris-Cl (pH=7.5), 100mM KCl, 1mM β-mercaptoethanol). The dialyzed protein underwent further purification steps. The protein was first applied to a Heparin-agarose column for 1 h. Afterwards, the column is extensively washed with dialysis buffer. The protein was then eluted from heparin column using an elution buffer (25mM Tris-Cl (pH=7.5), 500mM NaCl, 2mM β-mercaptoethanol). For further purification, the ultrapure protein fractions were applied to a Sephacryl column. The column was equilibrated in dialysis buffer and then 2ml protein concentrate was applied.Peakfractions were collected and analyzed via SDS PAGE. The ultra pure fractions were then dialyzed in dialysis buffer and stored at 4 °C for further analysis.

## REFERENCES

Bierhaus, A. et al. (2005) Understanding RAGE, the receptor for advanced glycation endproducts. J. Mol. Med. 83, 876-886

Bierhaus, A. & Nawroth, P.P. (2009) Multiple levels of regulation determine the role of the receptor for AGE (RAGE) as common soil in inflammation, immune responses and diabetes mellitus and its complications. Diabetologia. 52, 2251-2263

Demling N, Ehrhardt C, Kasper M, Laue M, Knels L, Rieber EP. Promotion of cell adherence and spreading: a novel function of RAGE, the highly selective differentiation marker of human alveolar epithelial type I cells. Cell and Tissue Researh.2006;323(3):475-488.

Fehrenbach H, Kasper M, Tschernig T, Shearman MS, Schuh D, Müller M. Receptor for advanced glycation end-products (RAGE) exhibits highly differential cellular and subcellular localisation in rat and human lung. Cellular and Molecular Biology. 1998;44(7):1147-1157.

He M, Kubo H, Ishizawa K, et al. The role of the receptor for advanced glycation end-products in lung fibrosis. American Journal of Physiology 2007;293(6): L1427-L1436

O'Driscoll M., Diseases associated with defective responses to DNA damage. Cold Spring Harb Perspect Biol, 2012. 4(12). Wielputz, M.O., et al., In vivo monitoring of cystic fibrosis-like lung disease in mice by volumetric compted tomography. Eur Respir J, 2011.38(5): p. 1060-70.

Dessing, M.C., et al., RAGE does not contribute to renal injury and damage upon ischemia/reperfusion-induced injury. J Innate Immun, 2012. 4(1):p. 80-5

Kumar V., Homeostatic interaction between Receptor for advanced glycation end products (RAGE) and Ataxia telangiectasia mutated (ATM) is essential for DNA repair, PhD Thesis, Ruprechts-Karls-University Heidelberg, 2015.Constein, R., et al., Characterization of a novel EGFP reporter mouse to monitor Cre recombination as demonstrated by a Tie2 Cre mouse line. Genesis, 2001. 30(1):36-44.

Jackson, S.P. ad J. Bartek, The DNA-damage response in human biology and disease. Nature, 2009.461 (7267):p. 1071-8-Ciccia, A. and S.J. Elledge, The DNA damage response: making it safe to play with knives. Mol Cell, 2010. 40(2): p. 179-204. Aparicio, T., R. Baer, and J. Gautier, DNA double-strand break repair pathway choice and cancer. DNA Repair (Amst), 2014. 19: 169-75.

Branzei, D. and M. Foiani, Regulation of DNA repair throughout the cell cycle. Nat Rev Mol Cell Biol, 2008.)(4): p. 297-308. Matsuoka, S. et al., ATM and ATR substrate analysis reveals extensive protein networks responsive to DNA damage. Science, 2007. 316(5828):p 1160-6.

Schmidt, A. M. et al. (1992). Isolation and characterization of two binding proteins for advanced glycosylation end products from bovine lung which are present on the endothelial cell surface. J Biol Chem, 267(21), 14987-14997

## SEQUENCES

SEQ ID NO: 1 Murine RAGE Q62151 of UniprotKB (Sequence version 2 (17 Feb 2016))
SEQ ID NO: 2 Phosphmomimetic mRAGE

MPAGTAARAWVLVLALWGAVAGGQNITARIGEPLVLSCKGAPKKPPQQLEWKLNTGRTEAWKVLSPQGGPWDSVARILPNGSLLLPATGIVDEGTFRCRATNRRGKEVKS
NYRVRVYQIPGKPEIVDPASELTASVPNKVGTCVSEGSYPAGTLSWHLDGKLLIPDGKETLVKEETRRHPETGLFTLRSELTVIPTQGGTHPTFSCSFSLGLPRRRPLNTAPI
QLRVREPGPPEGIQLLVEPEGGIVAPGGTVTLTCAISAQPPPQVHWIKDGAPLPLAPSPVLLLPEVGHEDEGTYSCVATHPSHGPQESPPVSIRVTETGDEGPAEGSVGESG
LGTLALALGILGGLGVVALLVGAILWRKRQPRREERKAPEEQEDEEERAELNQEEEAEMPENGAGGP

SEQ ID NO: 3 human RAGE Q15109 of UniprotKB (Sequence version 1 (01 Nov 1997))
SEQ ID NO: 4 Phosphmomimetic mRAGE (protein)

MAAGTAVGAWVLVLSLWGAVVGAQNITARIGEPLVLKCKGAPKKPPQRLEWKLNTGRTEAWKVLSPQGGGPWDSVARVLPNGSLFLPAVGIQDEGIFRCQAMNRNGKET
KSNYRVRVYQIPGKPEIVDSASELTAGVPNKVGTCVSEGSYPAGTLSWHLDGKPLVPNEKGVSVKEQTRRHPETGLFTLQSELMVTPARGGDPRPTFSCSFSPGLPRHRA
LRTAPIQPRVWEPVPLEEVQLVVEPEGGAVAPGGTVTLTCEVPAQPSPQIHWMKDGVPLPLPPSPVLILPEIGPQDQGTYSCVATHSSHGPQESRAVSISIIEPGEEGPTAGS
VGGSGLGTLALALGILGGLGTAALLIGVILWQRRQRRGEERKAPENQEEEEERAELNQEEEPEAGESSTGGP

SEQ ID NO: 5 Phosphmomimetic mRAGE (nucleotide)

ATGCCAGCGGGGACAGCAGCTAGAGCCTGGGTGCTGGTTCTTGCTCTATGGGGAGCTGTAGCTGGTGGTCAGAACATCACAGCCCGGATTGGAGAGCCACTTGTG
CTAAGCTGTAAGGGGGGCCCCTAAGAAGCCGCCCCAGCAGCTAGAATGGAAACTGAACACAGGAAGAACTGAAGCTTGGAAGGTCCTCTCTCCCCAGGGAGGCCCC
TGGGACAGCGTGGCTCGAATCCTCCCCAATGGTTCCCTCCTCCTTCCAGCCACTGGAATTGTCGATGAGGGGACTTTCCGGTGTCGGGCAACTAACAGGCGAGGG
AAGGAGGTCAAGTCCAACTACCGAGTCCGAGTCTACCAGATTCCTGGGAAGCCAGAAATTGTGGATCCTGCCTCTGAACTCACAGCCAGTGTCCCTAATAAGGTGG
GGACATGTGTGTCTGAGGGAAGCTACCCTGCAGGGACCCTTAGCTGGCACTTAGATGGGAAACTTCTGATTCCCGATGGCAAAGAAACACTCGTGAAGGAAGAGAC
CAGGAGACACCCTGAGACGGGACTCTTTACACTGCGGTCAGAGCTGACAGTGATCCCCACCCAAGGAGGAACCCATCCTACCTTCTCCTGCAGTTTCAGCCTGGGC
CTTCCCCGGCGCAGACCCCTGAACACAGCCCCCATCCAACTCCGAGTCAGGGAGCCTGGGCCTCCAGAGGGCATTCAGCTGTTGGTTGAGCCTGAAGGTGGAATA
GTCGCTCCTGGTGGGACTGTGACCTTGACCTGTGCCATCTCTGCCCAGCCCCCTCCTCAGGTCCACTGGATAAAGGATGGTGCACCCTTGCCCCTGGCTCCCAGCC
CTGTGCTGCTCCTCCCTGAGGTGGGGCACGAGGATGAGGGCACCTATAGCTGCGTGGCCACCCACCCTAGCCACGGACCTCAGGAAAGCCCTCCTGTCAGCATCA
GGGTCACAGAAACCGGCGATGAGGGGCCAGCTGAAGGCTCTGTGGGTGAGTCTGGGCTGGGTACGCTAGCCCTGGCCTTGGGGATCCTGGGAGGCCTGGGAGTA
GTAGCCCTGCTCGTCGGGGCTATCCTGTGGCGAAAACGACAACCCAGGCGTGAGGAGAGGAAGGCCCCGGAAgaaCAGGAGGATGAGGAGGAACGTGCAGAGCT
GAATCAGGAAGAGGAAGCGGAGATGCCAGAGAATGGTGCCGGGGGACCGTAA

SEQ ID NO: 6 Phosphmomimetic hRAGE (nucleotide)

ATGGCAGCCGGAACAGCAGTTGGAGCCTGGGTGCTGGTCCTCAGTCTGTGGGGGGCAGTAGTAGGTGCTCAAAACATCACAGCCCGGATTGGCGAGCCACTGGTG
CTGAAGTGTAAGGGGGGCCCCCAAGAAACCACCCCAGCGGCTGGAATGGAAACTGAACACAGGCCGGACAGAAGCTTGGAAGGTCCTGTCTCCCCAGGGAGGAGG
CCCCTGGGACAGTGTGGCTCGTGTCCTTCCCAACGGCTCCCTCTTCCTTCCGGCTGTCGGGATCCAGGATGAGGGGATTTTCCGGTGCCAGGCAATGAACAGGAAT
GGAAAGGAGACCAAGTCCAACTACCGAGTCCGTGTCTACCAGATTCCTGGGAAGCCAGAAATTGTAGATTCTGCCTCTGAACTCACGGCTGGTGTTCCCAATAAGGT
GGGGACATGTGTGTCAGAGGGAAGCTACCCTGCAGGGACTCTTAGCTGGCACTTGGATGGGAAGCCCCTGGTGCCTAATGAGAAGGGAGTATCTGTGAAGGAACA
GACCAGGAGACACCCTGAGACAGGGCTCTTCACACTGCAGTCGGAGCTAATGGTGACCCCAGCCCGGGGAGGAGATCCCCGTCCCACCTTCTCCTGTAGCTTCAG
CCCAGGCCTTCCCCGACACCGGGCCTTGCGCACAGCCCCCATCCAGCCCCGTGTCTGGGAGCCTGTGCCTCTGGAGGAGGTCCAATTGGTGGTGGAGCCAGAAG
GTGGAGCAGTAGCTCCTGGTGGAACCGTAACCCTGACCTGTGAAGTCCCTGCCCAGCCCTCTCCTCAAATCCACTGGATGAAGGATGGTGTGCCCTTGCCCCTTCC
CCCCAGCCCTGTGCTGATCCTCCCTGAGATAGGGCCTCAGGACCAGGGAACCTACAGCTGTGTGGCCACCCATTCCAGCCACGGGCCCCAGGAAAGCCGTGCTGT

CAGCATCAGCATCATCGAACCAGGCGAGGAGGGGCCAACTGCAGGCTCTGTGGGAGGATCAGGGCTGGGAACTCTAGCCCTGGCCCTGGGGATCCTGGGAGGCC

TGGGGACAGCCGCCCTGCTCATTGGGGTCATCTTGTGGCAAAGGCGGCAACGCCGAGGAGAGGAGAGGAAGGCCCCAGAAAACCAGGAGGAAGAGGAGGAGCG

TGCAGAACTGAATCAGgaGGAGGAACCTGAGGCAGGCGAGAGTAGTACTGGAGGGCCTCCGCGGATA

## Claims

1. A phosphomimetic RAGE protein comprising an amino acid sequence that is at least 90 % identical to a native mammalian RAGE isoform or fragment thereof wherein at least one serine present in the cytoplasmatic tail (CT) of the mammalian RAGE isoform is replaced by a phosphomimetic structure.

2. The phosphomimetic RAGE protein according to claim 1, wherein the native mammalian RAGE isoform or fragment thereof contains the amino acid sequence of amino acids 40 to 241 of SEQ ID NO: 1, and wherein the phosphomimetic structure is preferably selected from glutamic acid, aspartic acid.

3. The phosphomimetic RAGE according to claim 1 or 2, wherein the amino acid sequence is at least 95 % identical, preferably at least 98 % identical, more preferably at least 99 % identical to murine RAGE identified by SEQ ID NO: 1 or a fragment thereof, wherein Ser376 and/or Ser389 as defined in SEQ ID NO: 1 are replaced by phosphomimetic amino acid(s), in particular glutamic acids.

4. The phosphomimetic RAGE according to claim 3, comprising an amino acid sequence that is at least 95 % identical, preferably at least 98 % identical, more preferably at least 99 % identical to SEQ ID NO: 2.

5. The phosphomimetic RAGE according to claim 1 or 2, wherein the amino acid sequence is at least 95 % identical, preferably at least 98 % identical, more preferably at least 99 % identical to human RAGE identified by SEQ ID NO: 3 or a fragment thereof, wherein Ser391 as defined in SEQ ID NO: 3 is replaced by a phosphomimetic amino acid, in particular glutamic acid.

6. The phosphomimetic RAGE according to claim 5, comprising an amino acid sequence that is at least 95 % identical, preferably at least 98 % identical, more preferably at least 99 % identical to SEQ ID NO: 4.

7. A fusion protein of a mammalian RAGE protein comprising an amino acid sequence that is at least 90 % identical to a native mammalian RAGE isoform or fragment thereof according to claim 1 or 2, fused to at least one nuclear targeting structure.

8. The fusion protein according to claim 7, wherein the amino acid sequence of the mammalian RAGE is at least 95 % identical, preferably at least 98 % identical, more preferably at least 99 % identical to human RAGE identified by SEQ ID NO: 3 or murine RAGE identified by SEQ ID NO: 1 or a fragment thereof and the nuclear targeting structure is a nuclear localization sequence (NLS).

9. An isolated polynucleotide with a nucleic acid sequence encoding the phosphomimetic RAGE according to any of claims 1 to 6 or the fusion protein according to claim 7 or 8.

10. The isolated polynucleotide according to claim 9, with a nucleic acid sequence that is at least 95 % identical, preferably at least 98 % identical, more preferably at least 99 % identical to SEQ ID NO: 5 or 6.

11. A vector comprising a polynucleotide according to claim 9 or 10 and a promoter, wherein the promoter is preferably a tissue specific promoter, more preferably selected from the group consisting of a keratinocyte specific promoter, a liver specific promoter, a fibroblast specific promoter, or a macrophage specific promoter,

12. A composition comprising carrier and an active pharmaceutical ingredient, selected from a phosphomimetic RAGE protein according to any of claims 1 to 6, a fusion protein according to claim 7 or 8, and a vector according to claim 11, for use in the treatment or prevention of a disease in a patient, wherein the disease is preferably selected from a disease initiated by impaired DNA repair, a disease initiated by increased DNA damage or a disease initiated by increased senescence.

13. The composition for use according to claim 12, wherein the disease is a disease of the lung, nerve, kidney, blood vessels or lymphatic vessels, preferably the disease is selected from age associated diseases, liver fibrosis, diabetic complications, neurodegenerative diseases, radiation damage, ischemia reperfusion, stroke, myocardial infarction, tissue damage associated with renal failure and replacement therapy, septicemia, skin disorders such as impaired wound healing and psoriasis, pulmonary fibrosis, wherein the pulmonary fibrosis is in particular caused by smoking, pollution, diabetes, radiation, or chemotherapy.

**14.** The composition for use according to claim 12 or 13, wherein the carrier is an adeno associated virus (AAV) capsid, preferably a tissue specific AAV capsid.

**15.** A method of purification of a RAGE protein with a polyhistidine-tag, comprising the steps of:

- Providing a first solution comprising the RAGE protein with a polyhistidine-tag and contaminant proteins;
- Subjecting the first solution to a Ni-NTA chromatography and eluting a second solution containing the RAGE protein; and
- Subjecting the second solution to a heparin chromatography and eluting a third solution containing the RAGE protein.

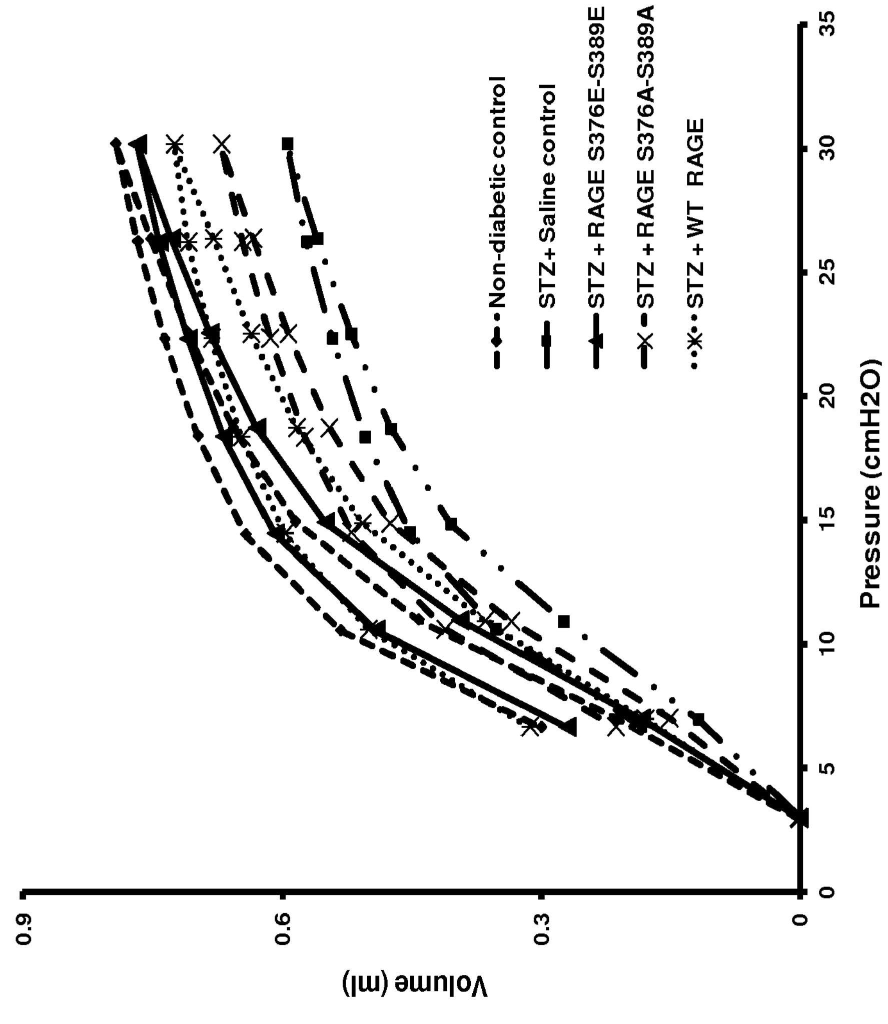
Volume (ml)
0
0.3
0.6
0.9
0
5
10
15
20
25
30
35
Pressure (cmH2O)
Non-diabetic control
STZ+ Saline control
STZ + RAGE S376E-S389E
STZ + RAGE S376A-S389A
STZ + WT RAGE

Fig. 1

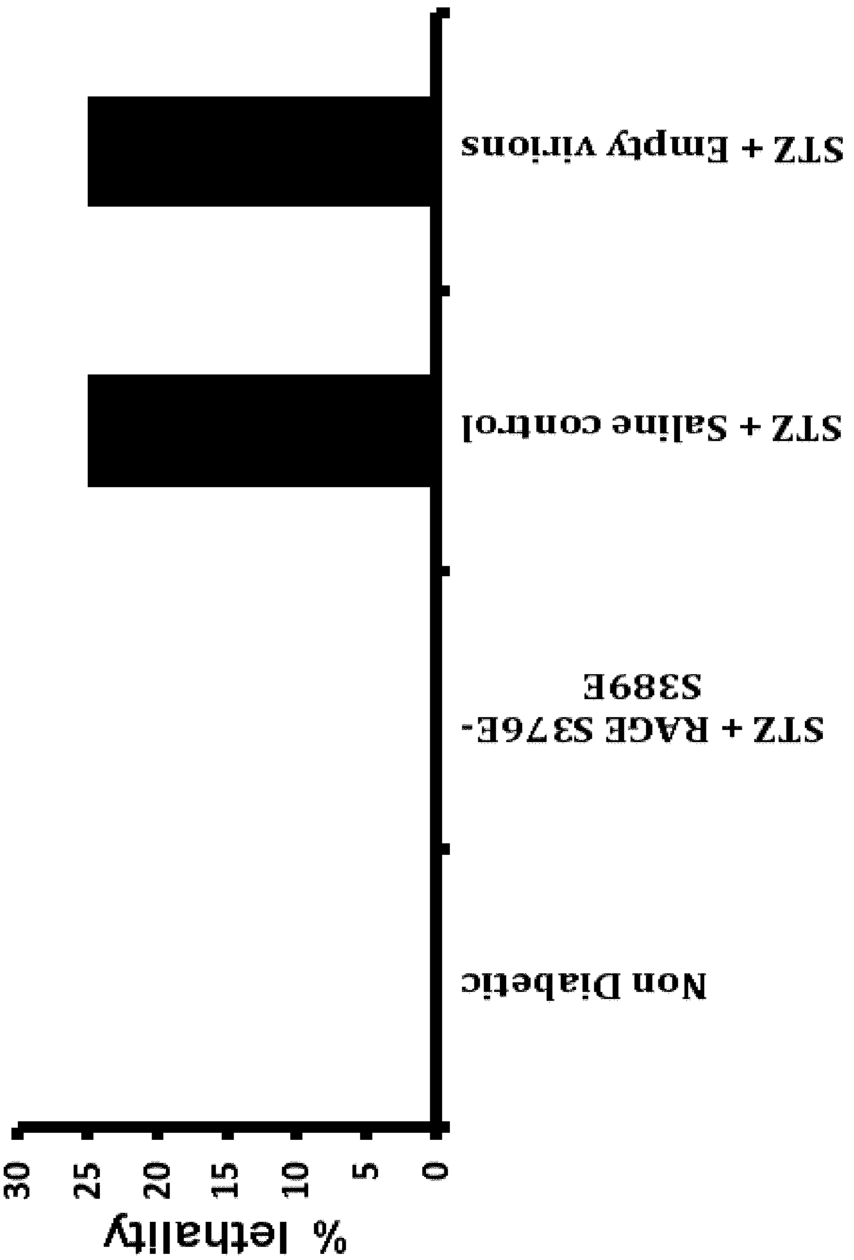
% lethality
0
5
10
15
20
25
30
Non Diabetic
STZ + RAGE S376E-S389E
STZ + Saline control
STZ + Empty virions

Fig. 2

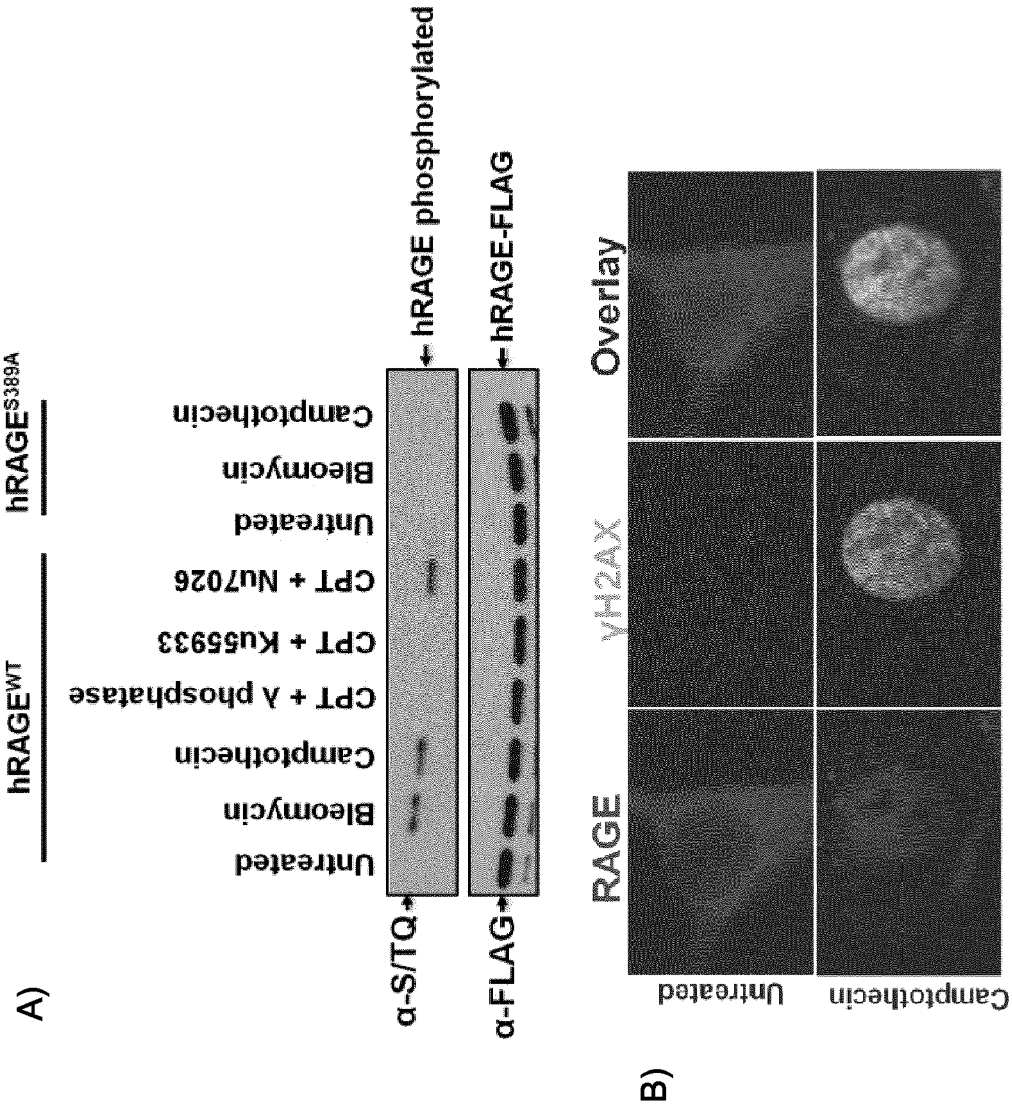
A)
hRAGE^WT
hRAGE^S389A
Untreated
Bleomycin
Camptothecin
CPT + λ phosphatase
CPT + Ku55933
CPT + Nu7026
Untreated
Bleomycin
Camptothecin
α-S/TQ
← hRAGE phosphorylated
α-FLAG
← hRAGE-FLAG
B)
RAGE
γH2AX
Overlay
Untreated
Camptothecin

Fig. 3

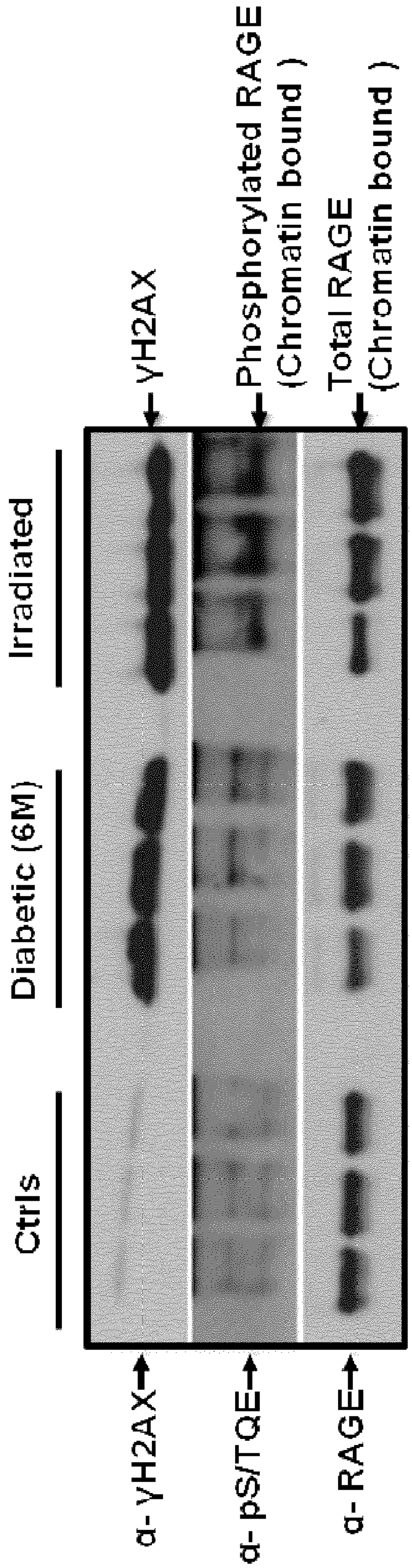
Ctrls
Diabetic (6M)
Irradiated
γH2AX
Phosphorylated RAGE
(Chromatin bound )
Total RAGE
(Chromatin bound )
α- γH2AX
α- pS/TQE
α- RAGE

Fig. 4

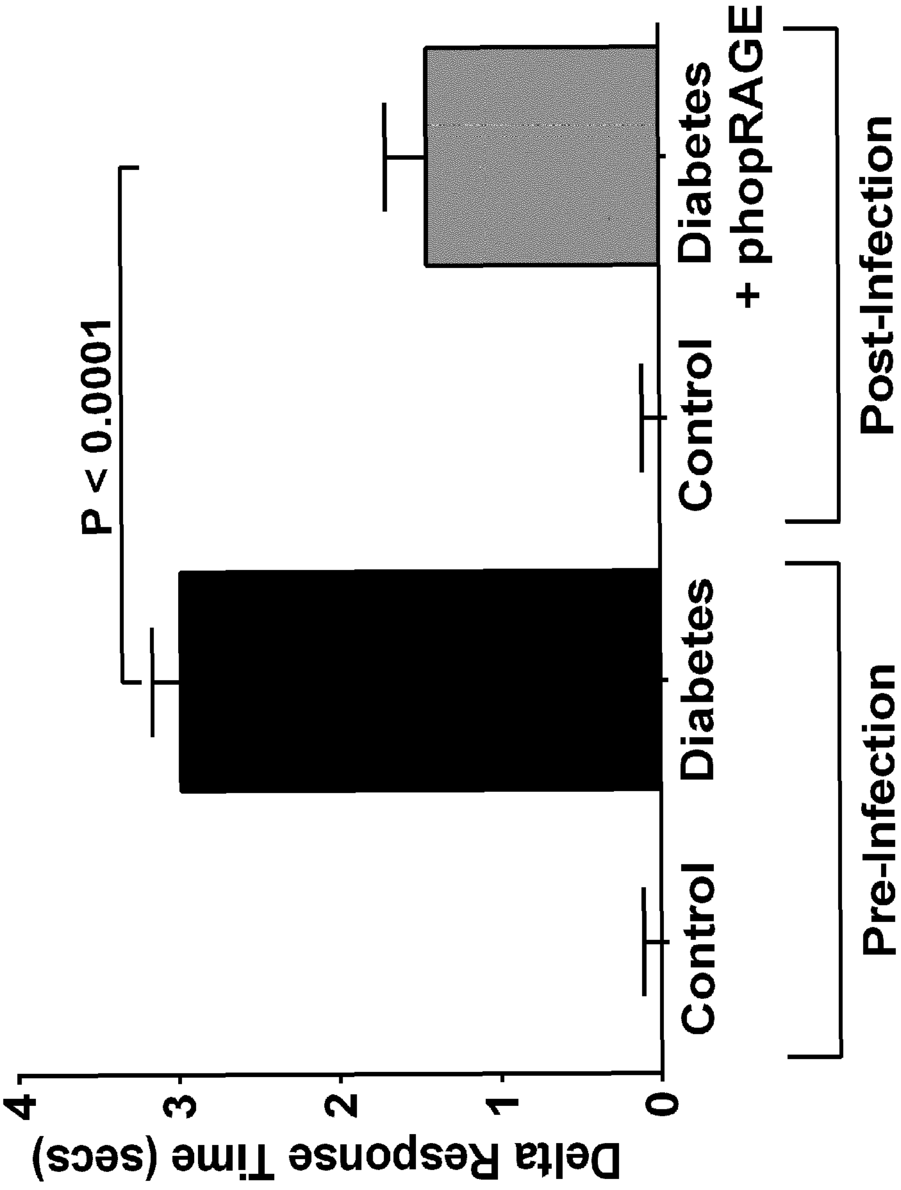
Delta Response Time (secs)
0
1
2
3
4
P < 0.0001
Control
Diabetes
Control
Diabetes + phopRAGE
Pre-Infection
Post-Infection Week 10

Fig. 5

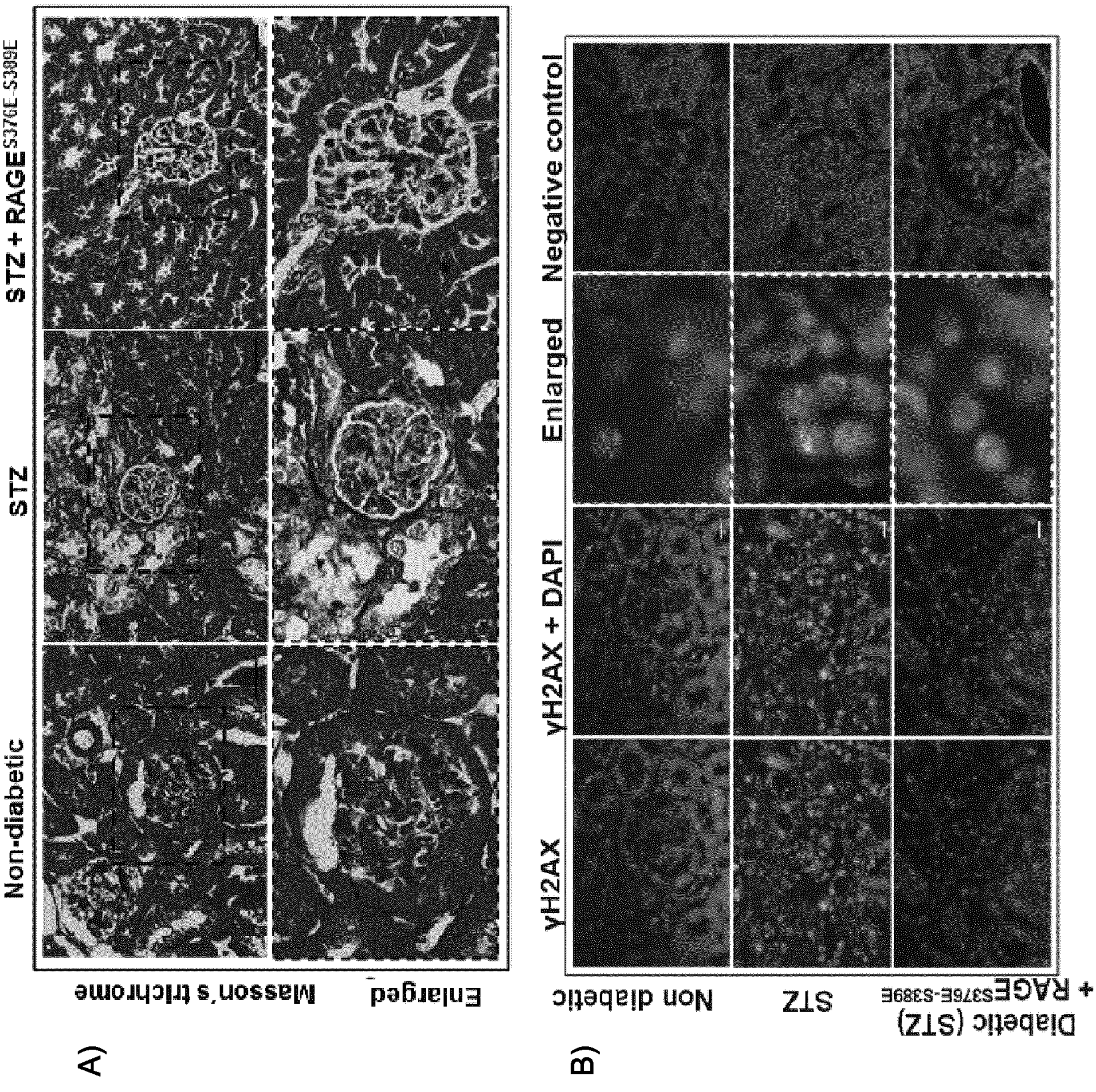

A)
Non-diabetic
STZ
STZ + RAGE S376E-S389E
Masson's trichrome
Enlarged
B)
γH2AX
γH2AX + DAPI
Enlarged
Negative control
Non diabetic
STZ
Diabetic (STZ) + RAGE S376E-S389E


Fig. 6

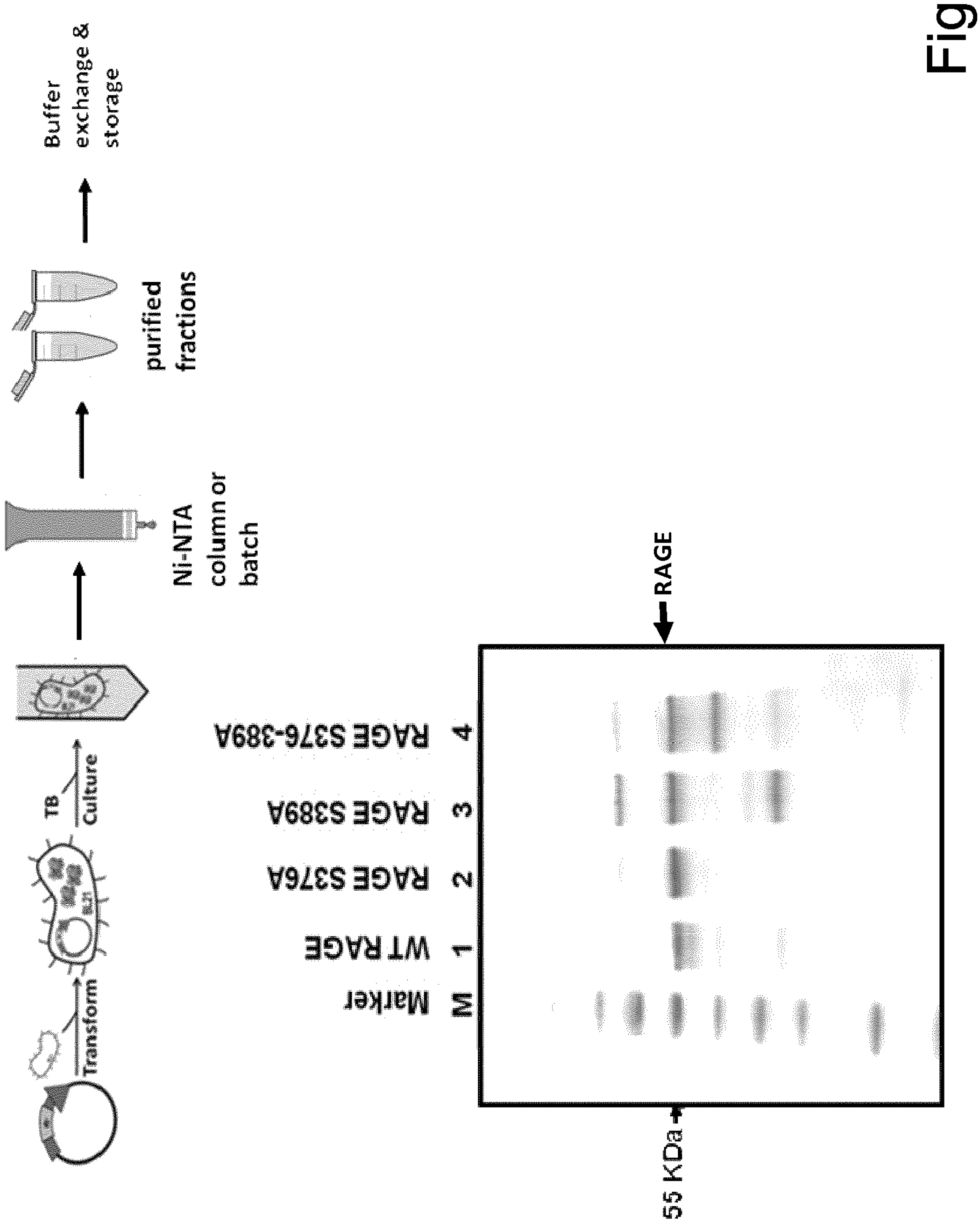
Transform
TB
Culture
Ni-NTA
column or
batch
purified
fractions
Buffer
exchange &
storage
M Marker
1 WT RAGE
2 RAGE S376A
3 RAGE S389A
4 RAGE S376-389A
RAGE
55 KDa

Fig. 7

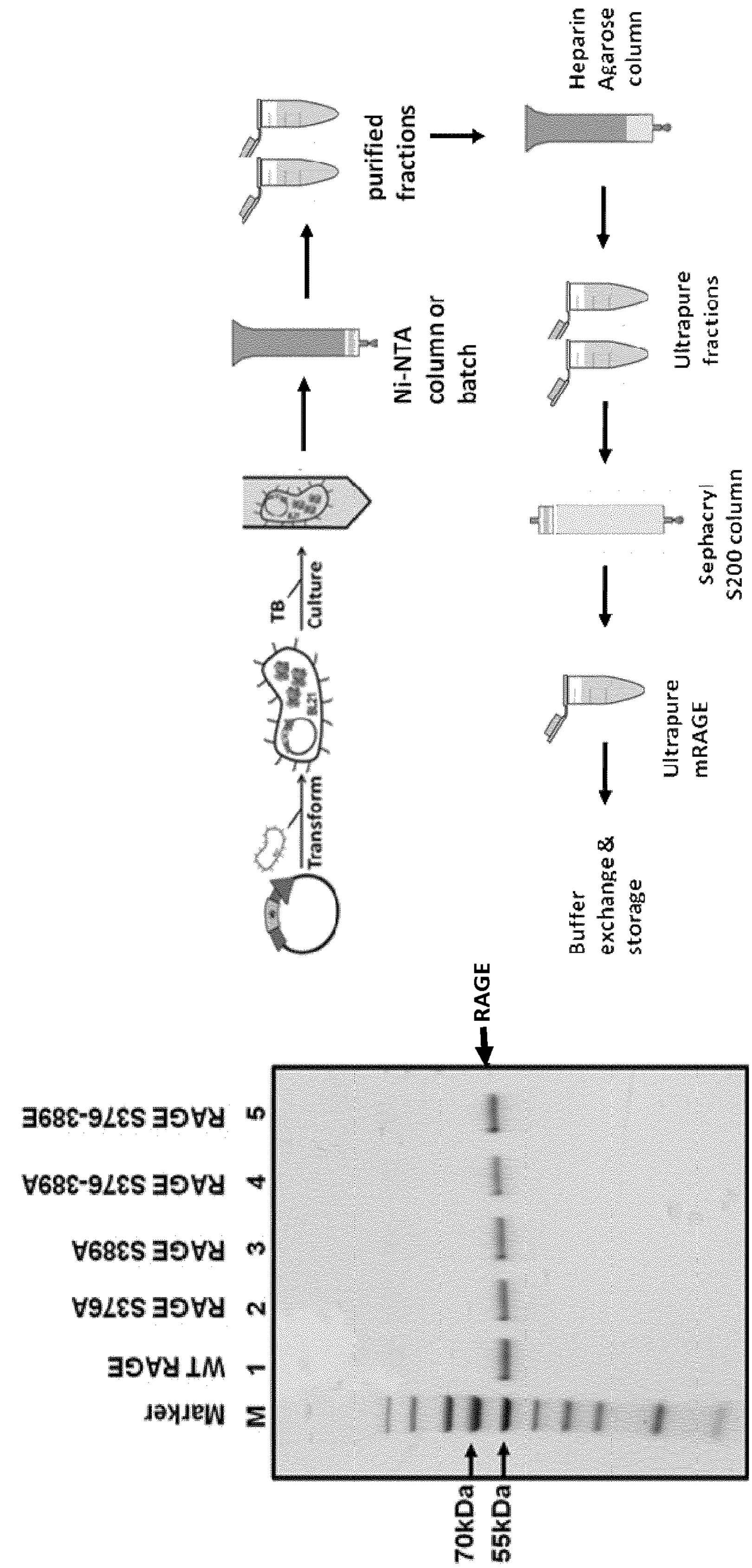
Transform
TB
Culture
Ni-NTA column or batch
purified fractions
Heparin Agarose column
Ultrapure fractions
Sephacryl S200 column
Ultrapure mRAGE
Buffer exchange & storage
RAGE
M Marker
1 WT RAGE
2 RAGE S376A
3 RAGE S389A
4 RAGE S376-389A
5 RAGE S376-389E
70kDa
55kDa

Fig. 8

Europäisches Patentamt
European Patent Office
Office européen des brevets

# EUROPEAN SEARCH REPORT

Application Number
EP 17 17 7679

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MASAKIYO SAKAGUCHI ET AL: "TIRAP, an Adaptor Protein for TLR2/4, Transduces a Signal from RAGE Phosphorylated upon Ligand Binding", PLOS ONE, vol. 6, no. 8, 1 August 2011 (2011-08-01), page e23132, XP055423659, DOI: 10.1371/journal.pone.0023132 * page 8 - page 9 * ----- | 1,5-13 | INV. C07K14/705 |
| X | WO 2009/108274 A2 (UNIV NORTH CAROLINA [US]; DIPRIMIO NINA [US]; SAMULSKI RICHARD JUDE [U) 3 September 2009 (2009-09-03) * paragraph [0163] - paragraph [0165] * ----- | 14 | |

TECHNICAL FIELDS SEARCHED (IPC)

C07K

~~The present search report has been drawn up for all claims~~

3

| Place of search | Date of completion of the search | Examiner |
|---|---|---|
| Munich | 16 November 2017 | Scheffzyk, Irmgard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

Application Number
EP 17 17 7679

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-14

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 17 17 7679

The Search Division considers that the present European patent application does not comply with the requirements of unity of invention and relates to several inventions or groups of inventions, namely:

1. claims: 1-14

   phosphomimetic RAGE protein

   ---

2. claim: 15

   method of purification of a RAGE protein

   ---

## ANNEX TO THE EUROPEAN SEARCH REPORT ON EUROPEAN PATENT APPLICATION NO. EP 17 17 7679

This annex lists the patent family members relating to the patent documents cited in the above-mentioned European search report. The members are as contained in the European Patent Office EDP file on
The European Patent Office is in no way liable for these particulars which are merely given for the purpose of information.

16-11-2017

| Patent document cited in search report | Publication date | Patent family member(s) | Publication date |
|---|---|---|---|
| WO 2009108274 A2 | 03-09-2009 | US 2009215879 A1 | 27-08-2009 |
| | | WO 2009108274 A2 | 03-09-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **NEEDLEMAN ; WUNSCH.** *J. Mol. Biol.,* 1970, vol. 48, 443-453
- **RICE.** *Trends Genet.,* 2000, vol. 16, 276-277
- **INNIS et al.** PCR: A Guide to Methods and Application. Academic Press, 1990
- Parvoviridae: The Viruses and Their Replication. **KENNETH I. BERNS.** Fields Virology. 1996
- **GOODMAN ; GILMAN'S et al.** The Pharmacological Basis of Therapeutics. McGraw-Hill Professional, 2001
- Science and Practice of Pharmacy. Lippincott Williams & Wilkins, 2003
- Pharmaceutical Dosage Forms and Drug Delivery Systems. Lippincott Williams & Wilkins, 1999
- Protein Purification. VCH Publishers, 1989
- **BIERHAUS, A. et al.** Understanding RAGE, the receptor for advanced glycation endproducts. *J. Mol. Med.,* 2005, vol. 83, 876-886
- **BIERHAUS, A. ; NAWROTH, P.P.** Multiple levels of regulation determine the role of the receptor for AGE (RAGE) as common soil in inflammation, immune responses and diabetes mellitus and its complications. *Diabetologia.,* 2009, vol. 52, 2251-2263
- **DEMLING N ; EHRHARDT C ; KASPER M ; LAUE M ; KNELS L ; RIEBER EP.** Promotion of cell adherence and spreading: a novel function of RAGE, the highly selective differentiation marker of human alveolar epithelial type I cells. *Cell and Tissue Researh,* 2006, vol. 323 (3), 475-488
- **FEHRENBACH H ; KASPER M ; TSCHERNIG T ; SHEARMAN MS ; SCHUH D ; MÜLLER M.** Receptor for advanced glycation endproducts (RAGE) exhibits highly differential cellular and subcellular localisation in rat and human lung. *Cellular and Molecular Biology.,* 1998, vol. 44 (7), 1147-1157
- **HE M ; KUBO H ; ISHIZAWA K et al.** The role of the receptor for advanced glycation end-products in lung fibrosis. *American Journal of Physiology,* 2007, vol. 293 (6), L1427-L1436
- **O'DRISCOLL M.** Diseases associated with defective responses to DNA damage. Cold Spring Harb Perspect Biol, 2012, vol. 4
- **WIELPUTZ, M.O. et al.** In vivo monitoring of cystic fibrosis-like lung disease in mice by volumetric compted tomography. *Eur Respir J,* 2011, vol. 38 (5), 1060-70
- **DESSING, M.C. et al.** RAGE does not contribute to renal injury and damage upon ischemia/reperfusion-induced injury. *J Innate Immun,* 2012, vol. 4 (1), 80-5
- Homeostatic interaction between Receptor for advanced glycation end products (RAGE) and Ataxia telangiectasia mutated (ATM) is essential for DNA repair. **KUMAR V.** PhD Thesis. Ruprechts-Karls-University Heidelberg, 2015
- **CONSTEIN, R. et al.** Characterization of a novel EGFP reporter mouse to monitor Cre recombination as demonstrated by a Tie2 Cre mouse line. *Genesis,* 2001, vol. 30 (1), 36-44
- **JACKSON, S.P. ; J. BARTEK.** The DNA-damage response in human biology and disease. *Nature,* 2009, vol. 461 (7267), 1071-8
- **CICCIA, A. ; S.J. ELLEDGE.** The DNA damage response: making it safe to play with knives. *Mol Cell,* 2010, vol. 40 (2), 179-204
- **APARICIO, T. ; R. BAER ; J. GAUTIER.** DNA double-strand break repair pathway choice and cancer. *DNA Repair (Amst),* 2014, vol. 19, 169-75
- **BRANZEI, D. ; M. FOIANI.** Regulation of DNA repair throughout the cell cycle. *Nat Rev Mol Cell Biol,* 2008, vol. 4, 297-308
- **MATSUOKA, S. et al.** ATM and ATR substrate analysis reveals extensive protein networks responsive to DNA damage. *Science,* 2007, vol. 316 (5828), 1160-6
- **SCHMIDT, A. M. et al.** Isolation and characterization of two binding proteins for advanced glycosylation end products from bovine lung which are present on the endothelial cell surface. *J Biol Chem,* 1992, vol. 267 (21), 14987-14997